# EUROPEAN PATENT APPLICATION

(11) **EP 4 394 796 A1**
(43) Date of publication of application: **03.07.2024**
(21) Application number: 23220790.2
(22) Date of filing: 29.12.2023
(51) Int. Cl.: G16H 50/20, G16H 50/70

(54) **MULTIMODAL CARDIO DISEASE STATE PREDICTIONS COMBINING ELECTROCARDIOGRAM, ECHOCARDIOGRAM, CLINICAL AND DEMOGRAPHICAL INFORMATION RELATING TO A PATIENT**

(30) Priority: 29.12.2022 US 202218091320
(71) Applicant: Tempus AI, Inc., Chicago, IL 60654 (US)
(72) Inventor: Nemani, Arun, Potomac, MD 20854 (US); Lee, Greg, Chicago, IL 60610 (US)
(74) Representative: Hofmann, Matthias

(57) **Abstract**

A method for configuring a single architecture deep learning model includes receiving parameters; configuring segments; and processing segment, combination and/or fully-connected outputs to generate modeling output. A computing system comprising: a processor; and a memory having stored thereon computer-executable instructions that, when executed, cause the computing system to: receive parameters; configure segments; and process segment, combination and/or fully-connected outputs to generate modeling output. A non-transitory computer-readable medium having stored thereon computer-executable instructions that, when executed by one or more processors, cause a computer to: receive parameters; configure segments; and process segment, combination and/or fully-connected outputs to generate modeling output.

## Description

### BACKGROUND OF THE DISCLOSURE

The field of the disclosure is directed to multimodal cardio disease state predictions combining electrocardiogram (ECG), echocardiogram (echo), clinical and demographical information relating to a patient (e.g., electronic health records (EHR)), and more specifically, to methods and systems for configuring and using of one or more single architecture (ML) models.

Conventional techniques are not able to diagnose clinical conditions like atrial fibrillation (AF) early or quickly, which leads to detrimental patient outcomes. Analysis of an ECG is used to diagnose and treat many different heart diseases but are unable to forecast whether a condition such as AF will develop. Conventional techniques for attempting forecasting may include training a plurality of machine learning models wherein each one is separately trained to perform a particular predictive technique. For example, existing techniques require training and operation of a first model for processing ECG data, a second model for processing electronic health records, etc. However, this leads to several problems. First, an ensemble modeling approach may have the risk of error propagation. Each model presents its own set of uncertainties, which are directly passed onto uncertainties in downstream models. Second, the different models must be separately trained, maintained, and deployed, which leads to significant overhead in a production computing environment. For example, users may train a new model with more electronic healthcare record (EHR) features and merge already trained ECG-only feature models. However, the upstream ECG-only model is not able to map any non-linear interactions with additional EHR features since they are completely different models.

Furthermore, any other datasets that include imaging datasets, unstructured datasets, or potential future genomic datasets would require preprocessing and modeling outside of this framework. This approach presents several drawbacks. First, a single loss function may not effectively be used for all features in a traditional sense (ECG, structured, and imaging data for example), since modeling is decoupled into ECG only via the neural network approach, and then ensembled with alternative modeling approaches that deal with clinical features. These ensembled modeling approaches have their own underlying implementation differences, different training parameters, and loss functions, which yield suboptimal modeling results. Second, the conventional techniques are unable to use ECG features and clinical features in modeling frameworks that are not classification or regression based such as survival modeling, thus limiting the scope of modeling outputs for disease onset predictions. Unlike neural networks, alternative modeling methods often have constraints on the number of parameters that can be trained, presenting a challenge in improving the robustness of model performance towards disease predictions.

Thus, improved techniques for predicting cardiac states that can use multiple input types together are needed.

### SUMMARY OF THE DISCLOSURE

In one aspect, a computer-implemented method for configuring a single architecture deep learning model to process a plurality of dissimilar input feature modalities includes (i) receiving predefined configuration parameters corresponding to three or more segments, each respective segment corresponding to a different respective input feature modality, each respective segment including one or more respective preprocessing layers, and each respective segment including a respective plurality of isolated processing layers; (ii) configuring each segment based on the predefined configuration parameters; (iii) for each respective segment:
preprocessing the dissimilar input feature modalities using the respective preprocessing layers to generate respective preprocessing outputs, and processing the respective preprocessing outputs via the respective plurality of isolated processing layers to generate respective segment outputs, wherein the processing includes generating normalized output compatible with one or more other respective plurality of isolated processing layers; and (iv) processing the respective segment outputs via one or more combination layers to generate a combination output, wherein the combination output implements one or more loss functions, and wherein the combination output has an output type.

In another aspect, a computing system for configuring a single architecture deep learning model to process a plurality of dissimilar input feature modalities includes one or more processors; and one or more memories having stored thereon computer-executable instructions that, when executed, cause the computing system to: (i) receive predefined configuration parameters corresponding to three or more segments, each respective segment corresponding to a different respective input feature modality, each respective segment including one or more respective preprocessing layers, and each respective segment including a respective plurality of isolated processing layers; (ii) configure each segment based on the predefined configuration parameters; (iii) for each respective segment: preprocess the dissimilar input feature modalities using the respective preprocessing layers to generate respective preprocessing outputs, and process the respective preprocessing outputs via the respective plurality of isolated processing layers to generate respective segment outputs, wherein the processing includes generating normalized output compatible with one or more other respective plurality of isolated processing layers; and (iv) process the respective segment outputs via one or more combination layers to generate a combination output, wherein the combination output implements one or more loss functions, and wherein the combination output has an output type.

In yet another aspect, a non-transitory computer-readable medium includes computer-executable instructions that, when executed by one or more processors, cause a computer to: (i) receive predefined configuration parameters corresponding to three or more segments, each respective segment corresponding to a different respective input feature modality, each respective segment including one or more respective preprocessing layers, and each respective segment including a respective plurality of isolated processing layers; (ii) configure each segment based on the predefined configuration parameters; (iii) for each respective segment: preprocess the dissimilar input feature modalities using the respective preprocessing layers to generate respective preprocessing outputs, and process the respective preprocessing outputs via the respective plurality of isolated processing layers to generate respective segment outputs, wherein the processing includes generating normalized output compatible with one or more other respective plurality of isolated processing layers; (iv) process the respective segment outputs via one or more combination layers to generate a combination output, wherein the combination output implements one or more loss functions, and wherein the combination output has an output type.

### BRIEF DESCRIPTION OF THE SEVERAL VIEWS OF THE DRAWINGS

In accordance with common practice, the various features illustrated in the drawings may not be drawn to scale. The illustrations presented herein are not meant to be actual views of any particular method, device, or system, but are merely idealized representations that are employed to describe various aspects of the disclosure. Accordingly, the dimensions of the various features may be arbitrarily expanded or reduced for clarity. In addition, some of the drawings may be simplified for clarity. Thus, the drawings may not depict all of the components of a given apparatus (e.g., device) or method. In addition, like reference numerals may be used to denote like features throughout the specification and figures.
FIG. 1A depicts an exemplary computing system for configuring a single architecture deep learning model to process predicting a plurality of dissimilar input feature modalities, according to some aspects.
FIG. 1B depicts exemplary hardware devices that may be used in some aspects of the system of FIG. 1A, according to some aspects.
FIG. 2 depicts exemplary raw ECG voltage input data, according to some aspects.
FIG. 3 depicts an exemplary configurable single architecture machine learning model, according to some aspects.
FIG. 4 depicts an exemplary configurable single architecture machine learning model for generating a predicted cardiac amyloid risk score, according to some aspects.
FIG. 5 depicts a loss function tailored for regression, according to some aspects.
FIG. 6 depicts a deep learning network architecture for multi-class classification, according to some aspects.
FIG. 7 depicts an exemplary computer-implemented method, according to some aspects.

In the following detailed description, reference is made to the accompanying drawings which form a part hereof, and in which is shown by way of illustration, specific aspects in which the disclosure may be practiced. These aspects are described in sufficient detail to enable those of ordinary skill in the art to practice the disclosure. It should be understood, however, that the detailed description and the specific examples, while indicating examples of aspects of the disclosure, are given by way of illustration only and not by way of limitation. From this disclosure, various substitutions, modifications, additions, rearrangements, or combinations thereof within the scope of the disclosure may be made and will become apparent to those of ordinary skill in the art.

### DETAILED DESCRIPTION OF THE DISCLOSURE

The various aspects of the subject disclosure are now described with reference to the drawings, wherein like reference numerals correspond to similar elements throughout the several views. It should be understood, however, that the drawings and detailed description hereafter relating thereto are not intended to limit the claimed subject matter to the particular form disclosed. Rather, the intention is to cover all modifications, equivalents, and alternatives falling within the spirit and scope of the claimed subject matter.

### Overview

The present application is directed to, *inter alia,* methods and systems for multimodal cardio disease state predictions for configuring and using a single architecture ML model (e.g., a deep learning model) to process a plurality of dissimilar input feature modalities, according to some aspects.

Herein, "dissimilar" modalities include modalities that are not compatible to analyze within a specific neural network layer due to the differences in input shapes. For example, a single ECG lead may have a shape of (1, 5000) where the first number pertains to the number of ECGs and the second pertains to the length of the waveform for that single lead. An EHR feature may have an input shape of (1, 3), where the first number pertains to the number of EHR records for that patient encounter and the second number pertains to the length of EHR features (ex. QRS interval length, BMI, smoking status).

12-lead electrocardiogram may include a I Lateral lead (also referred to as a I lead), a II Inferior lead (also referred to as a II lead), a III Inferior lead (also referred to as a III lead), an aVR lead, an aVL Lateral lead (also referred to as an aVL lead), an aVF Inferior lead (also referred to as an aVF lead), a V1 Septal lead (also referred to as a V1 lead), a V2 Septal lead (also referred to as a V2 lead), a V3 Anterior lead (also referred to as a V3 lead), a V4 Anterior lead (also referred to as a V4 lead), a V5 Lateral lead (also referred to as a V5 lead), and a V6 Lateral lead (also referred to as a V6 lead). aVR, aVL and aVF are the augmented unipolar limb leads, introduced by Emanuel Goldberger.

### Exemplary Computing System

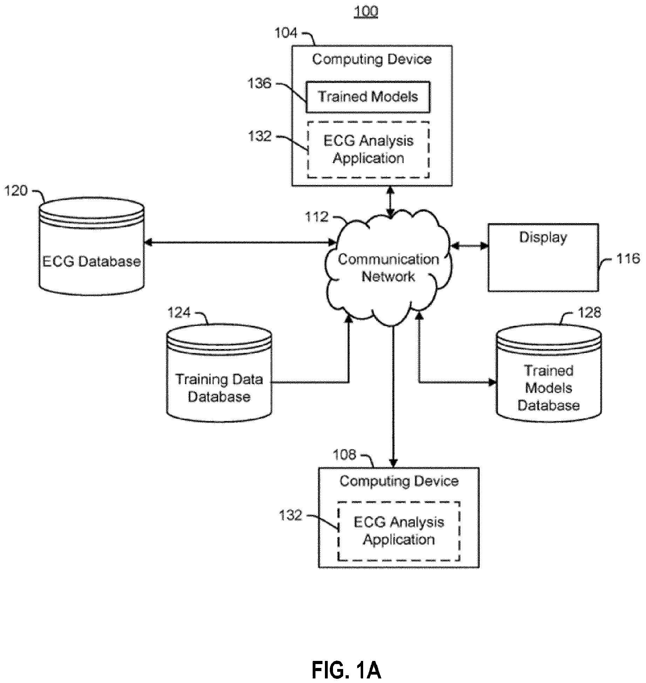

FIG. 1A depicts an exemplary computing example of a system 100 for configuring one or more machine learning models (e.g., one or more deep learning models) to process a plurality of dissimilar input feature modalities, according to some aspects. In some aspects, the system 100 may include a computing device 104, a secondary computing device 108, and/or a display 116. In some aspects, the system 100 may include an ECG database 120, a training data database 124, and/or a trained models database 128. In some aspects, the computing device 104 can be in communication with the secondary computing device 108, the display 116, the ECG database 120, the training data database 124, and/or the trained models database 128 via an electronic communication network 112. The system 100 may be used to train and use one or more ML models (e.g., a deep learning model) using single architecture techniques discussed herein.

As shown in FIG. 1A, the computing device 104 may receive ECG data, such as 12-lead ECG data, which may be used for example, to generate a cardiac risk score (e.g., AF, AS, CA, and/or SP) based on the ECG data using one or more single architecture models. In some aspects, the risk score can indicate a predicted risk of a patient developing the cardiac event within a predetermined time period from when the ECG was taken (e.g., three months, six months, one year, five years, ten years, etc.). In some aspects, the computing device 104 can execute at least a portion of an ECG analysis application 132 to automatically generate the risk score.

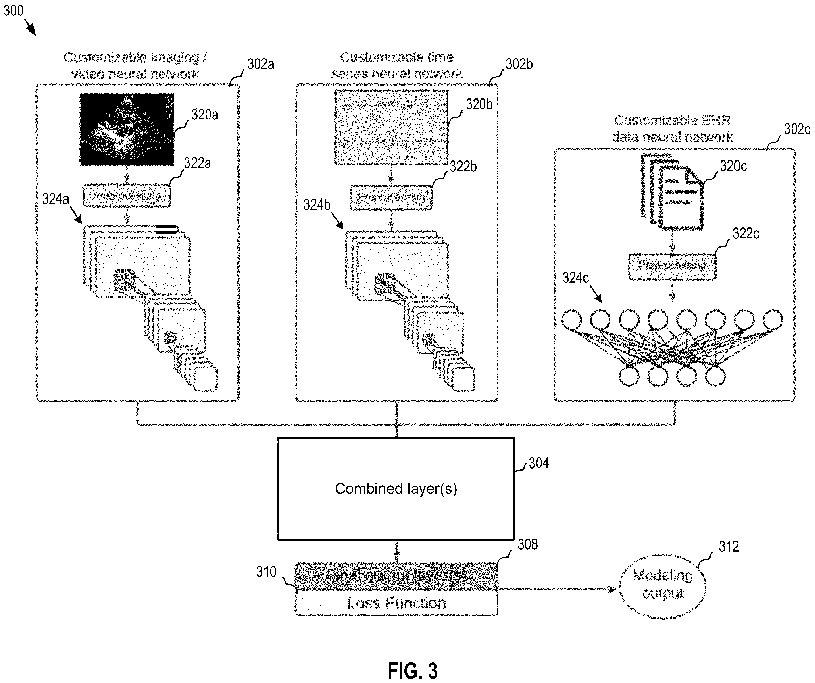

The system 100 may receive one or more predefined model configuration parameters from a user of the system 100. For example, the configuration parameters may be submitted to the system by a user of the display 116. The user may access a graphical user interface application displayed in the display 116 that enables the user to enter one or more configuration parameters. For example, the user may enter, or choose from among, a number of model segments, wherein each model segment corresponds to a different respective input feature modality. In this way, the user may provide configuration parameters that specify multiple dissimilar input modalities, each corresponding to a different respective modeling segment. For example, FIG. 3 depicts an example single architecture having three dissimilar input modalities. In some aspects, the configuration parameters may be included in a configuration file received by the system 100 (e.g., a JSON file, a YAML file, an XML file, etc.).

Such a configuration file may include, for example, a switch value enabling the multimodal modeling to be selectively enabled or disabled; a list of preprocessor classes; a mapping of columns to one or more types (e.g., numeric, categorical, etc.). In some aspects, the file may include a list of columns to ignore. In some aspects, the configuration file may include one or more predefined preprocessors. For example, the predefined preprocessors may include numeric preprocessors (e.g., a standard scalar function), categorical preprocessors (e.g., one hot encoder functions), etc. The configuration file may include information regarding hidden layers. For example, the configuration file may define an auxiliary hidden layer having a dense batch normalization layer, a given depth (e.g., 4 layers), a number of units for the respective layers (e.g., 128, 64, 8 and 1) and a list of activation functions (e.g., ReLU, SWISH, MISH, sigmoid) with respect to each of the layers, wherein ReLU is a rectified linear activation function, such as a binary/piecewise linear function. In some aspects, other activation functions may be used, such as linear, sigmoid, tanh, and leaky ReLU activation functions. The configuration file is one way that a user of the system can easily provide configuration parameters for modeling any disease and a variety of modeling endpoints. As mentioned above, a significant drawback in conventional techniques is that different models may present uncertainties within each model, which is propagated to downstream models during ensembling.

Another drawback is that there may be non-linear associations between ECG features and all of the other EHR features which may not be mapped correctly if modeled separately.

Another drawback is that it is quite challenging to combine individual models towards non regression or non-classification type models. The present techniques advantageously enable heterogeneous features to be used together (e.g., ECG features and EHR features) to produce models that can effectively predict the risk of a cardiac disease onset. For example, ECG features and clinical features (e.g., EHR) may be processed by a configurable single architecture model (e.g., an artificial neural network, deep learning model, etc.). The present techniques are disease, modeling output, and cloud infrastructure agnostic, and thus can apply towards a wide variety of future endpoints, that include but are not limited to, stroke, rare cardiac diseases, heart failure, etc.

The system 100 may be used to train and/or operate one or more sets of isolated layers that a parameterized, configured and generated by the configuration parameters. For example, the system 100 may create one or more models to generate a risk score to provide physicians with a recommendation to consider additional cardiac monitoring for patients who are most likely to experience atrial fibrillation, atrial flutter, or another relevant condition within the predetermined time period. Example sets of isolated layers are discussed below. In some aspects, the training of models may be defined based on the configuration parameters. In some aspects, the training of models may be defined based on the system 100 automatically detecting certain features regarding inputs (e.g., auto-bucketing ). As noted, conventional techniques do not handle the case of a multimodal modeling strategy wherein inputs include different model segments. The present techniques allow such disparate feature sets to be married into a single feature set and processed by a single ML architecture. In some aspects, the present techniques may be used to create a single feature set that feeds into an artificial neural network, and store the features separately. An example of this, discussed below, is an ECG database, and an EHR table set that are feature sets stored in a different manner.

In some modeling aspects, the present techniques may combine a single ECG waveform with a respective patient's EHR features during the time of the ECG into a single feature set and feed this combination into a single artificial neural network. In that case, ECG sub features may go into an ECG sub model, while EHR subfeatures go into an EHR sub model. The respective results of these sub models may be later combined for a prediction.

This enables a single ML architecture to map complex non-linear relationships between the various different multi-modal features for a given independent input, resulting in significant improvements in model performance and producing accurate and informative analytical results.

The present techniques enable robust combination of multimodal models that is not possible conventionally, in an entirely configurable manner. For example, an existing artificial neural network model may process ECG waveform to predict AF. Using the present techniques, the system 100 may enrich the model with additional multimodal data feature (e.g., patient age, sex, smoking history, hypertension history, vitals, BMI, etc.) to realize improvements in predictive power. As discussed below, the present techniques enable the addition, deletion and reconfiguration of additional/different multimodal models, and/or reconfigure the combination of models already part of the combination. Those of ordinary skill in the art will realize that some modalities may not increase the accuracy of some models and that those modalities need to be selected to optimize the configurable architecture's overall performance.

Nonetheless, the present techniques represent a breakthrough in the ability to combine multimodal features to perform cardiac state modeling in a single ML architecture. In particular, the system 100 may configure the model as a single network capable of receiving and processing wave-based data (e.g., ECG) in addition to numerical features, categorical features, Boolean features, etc. The single network architecture is especially significant, considering that a single loss function and single objective function may then be used to process all of the multimodal data. Similarly, in the classification context, a single output may be produced.

The system 100 includes instructions that enable a user to selectively configure one or more model segments. The configuration may include selecting preexisting model segments corresponding to different respective input modalities, as discussed, and further, different preprocessing and sets of models within each segment. For example, the system 100 may include instructions that enable a model to receive EHR features, preprocess the EHR features, scale them as needed for an artificial neural network. The system 100 may include further instructions that enable a model to receive ECG features, preprocess the ECG features, and scale them as needed for an artificial neural network. The system 100 may include instructions for combining the received EHR and ECG features into a single artificial neural network architecture.

The system 100 may configure the one or more models for different modeling paradigms. For example, system 100 may configure the models differently based on whether the modeling involves regression analysis, multi-classification, binary classification, etc. The user may define the modeling paradigm via the configuration parameters, and the system 100 may configure the model based on the configuration parameters. For example, when the user wants to model a problem that requires a regression analysis (e.g., predicting the thickness of the septal wall in a patient's heart) the user may simply pass a parameter of ("network type" : RegressionModel). Those of ordinary skill in the art will appreciate that one strength of a regression model is a numerical scale as an output, rather than a classification. In some aspects, the parameter may correspond to a class name of an existing class or one that the user references via a coding platform.

In the case of a multi-classification problem, the user may have a plurality of existing models, each of which separately process input features (e.g., ECGs, echocardiograms, EHR to predict 5-10 different disease states). The user may use the present techniques to refactor such a sprawling code base into a single network capable of predicting the probability of each conditions (e.g., AF, heart failure, etc.) in a single inferential loop. By doing so, the user may advantageously avoid deploying many (e.g., 10 or more) additional networks. This may result in a very large reduction in computational resources, due to reduced memory and computational output (e.g., CPU cycles).

In some aspects, the ECG data may be indicative or not indicative of a heart condition based on cardiological standards. For example, the ECG data may be indicative of a fast heartbeat including heart rate and heart rhythm. The system 100 may predict a risk score indicative that the patient will suffer from the cardiac condition (e.g., AF) based on ECG data that is not indicative of a given heart condition (e.g., fast heartbeat). In this way, the system may detect patients at risk for one or more conditions even when the ECG data appears "healthy" based on cardiological standards. The system 100 may predict a risk score indicative that the patient will suffer from the condition (e.g., AF) based on ECG data that is indicative of a heart condition (e.g., fast heartbeat). In this way, the system 100 may detect patients at risk for one or more conditions when the ECG data indicates the presence of a different condition.

The ECG analysis application 132 can be included in the secondary computing device 108 that can be included in the system 100 and/or on the computing device 104. The computing device 104 can be in communication with the secondary computing device 108. The computing device 104 and/or the secondary computing device 108 may also be in communication with a display 116 that can be included in the system 100 over the communication network 112. In some aspects, the computing device 104 and/or the secondary computing device 108 can cause the display 116 to present one or more AF risk scores and/or reports generated by the ECG analysis application 132.

The communication network 112 can facilitate communication between the computing device 104 and the secondary computing device 108. In some aspects, the communication network 112 can be any suitable communication network or combination of communication networks. For example, the communication network 112 may include a Wi-Fi network (which may include one or more wireless routers, one or more switches, etc.), a peer-to-peer network (e.g., a Bluetooth network), a cellular network (e.g., a 3G network, a 4G network, a 5G network, etc., complying with any suitable standard, such as CDMA, GSM, LTE, LTE Advanced, WiMAX, etc.), a wired network, etc. In some aspects, the communication network 112 can be a local area network, a wide area network, a public network (e.g., the Internet), a private or semi-private network (e.g., a corporate or university intranet), any other suitable type of network, or any suitable combination of networks. Communications links shown in FIG. 1A can each be any suitable communications link or combination of communications links, such as wired links, fiber optic links, Wi-Fi links, Bluetooth links, cellular links, etc.

The ECG database 120 may include a number of ECGs. In some aspects, the ECGs may include 12-lead ECGs. Each ECG may include a number of voltage measurements taken at regular intervals (e.g., at a rate of 250 HZ, 500 Hz, 1000 Hz, etc.) over a predetermined time period (e.g., 5 seconds, 10 seconds, 15 seconds, 30 seconds, 60 seconds, etc.) for each lead. In some instances, the number of leads may vary (e.g., from 1-12) and the respective sampling rates and time periods may be different for each lead. In some aspects, the ECG may include a single lead. In some aspects, the ECG database 120 may include one or more AF risk scores generated by the ECG analysis application 132.

The training data database 124 may include a number of ECGs and clinical data. In some aspects, the clinical data may include outcome data, such as whether or not a patient developed AF in a time period following the day that the ECG was taken. Exemplary time periods may include 1 month, 2 months, 3 months, 4 months, 5 months, 6 months, 7 months, 8 months, 9 months, 10 months, 11 months 12 months, 1 year, 2 years, 3 years, 4 years, 5 years, 6 years, 7 years, 8 years, 9 years, or 10 years. The ECGs and clinical data can be used for training a model to generate AF risk scores. In some aspects, the training data database 124 may include multi-lead ECGs taken over a period of time (such as ten seconds) and corresponding clinical data. In some aspects, the trained models database 128 may include a number of trained models that can receive raw ECGs and output AF risk scores. In other aspects, a digital image or digital map generated from signals from one or more lead for an ECG (e.g., ECG imaging) may be used. In some aspects, trained models 136 can be stored in the computing device 104.

### Exemplary Computing Devices

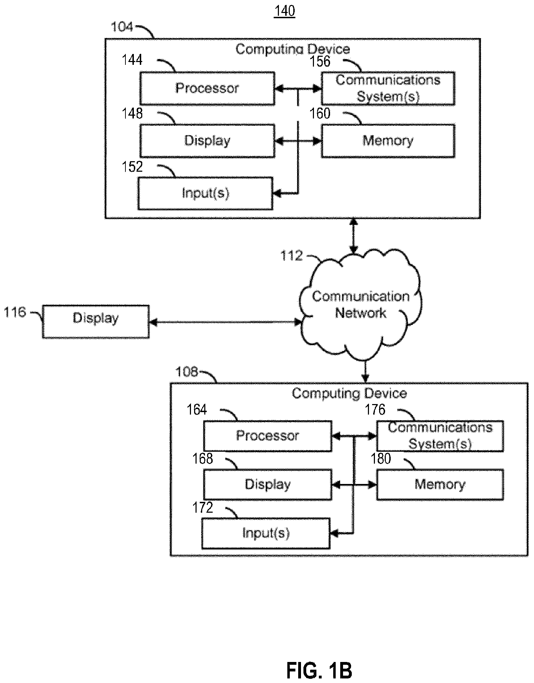

FIG. 1B is an example of exemplary computing devices that can be used in some aspects of the system 100, including a computing device 104 and a computing device 108. The computing device 104 may include a processor 144, a display 148, one or more input(s) 152, one or more communication system(s) 156, and a memory 160. The processor 144 may be any suitable hardware processor or combination of processors, such as a central processing unit ("CPU"), a graphics processing unit ("GPU"), etc. The processor may access and/or execute one or more sets of computer-executable instructions in the memory 160.

In some aspects, the display 148 may present a graphical user interface. In some aspects, the display 148 can be implemented using any suitable display devices, such as a computer monitor, a touchscreen, a television, etc. In some aspects, the input(s) 152 of the computing device 104 may include indicators, sensors, actuatable buttons, a keyboard, a mouse, a graphical user interface, a touch-screen display, etc.

In some aspects, the communication system(s) 156 may include any suitable hardware, firmware, and/or software for communicating with the other systems, over any suitable communication networks. For example, the communication system 156 may include one or more transceivers, one or more communication chips and/or chip sets, etc. In a more particular example, communication system 156 may include hardware, firmware, and/or software that can be used to establish a coaxial connection, a fiber optic connection, an Ethernet connection, a USB connection, a Wi-Fi connection, a Bluetooth connection, a cellular connection, etc. In some aspects, the communication system 156 allows the computing device 104 to communicate with the secondary computing device 108.

In some aspects, the memory 160 may include any suitable storage device or devices that can be used to store instructions, values, etc., that can be used, for example, by the processor 144 to present content using display 148, to communicate with the secondary computing device 108 via communications system(s) 156, etc. The memory 160 may include any suitable volatile memory, non-volatile memory, storage, or any suitable combination thereof. For example, the memory 160 may include RAM, ROM, EEPROM, one or more flash drives, one or more hard disks, one or more solid state drives, one or more optical drives, etc. In some aspects, the memory 160 can have encoded thereon a computer program for controlling operation of computing device 104 (or secondary computing device 108). In such aspects, the processor 144 can execute at least a portion of the computer program to present content (e.g., user interfaces, images, graphics, tables, reports, etc.), receive content from the secondary computing device 108, transmit information to the secondary computing device 108, etc.

The secondary computing device 108 may include a processor 164, a display 168, one or more input(s) 172, one or more communication system(s) 176, and a memory 180. The processor 164 can be any suitable hardware processor or combination of processors, such as a central processing unit ("CPU"), a graphics processing unit ("GPU"), etc., which can execute a program, which may include the processes described below.

In some aspects, the display 168 can present a graphical user interface. In some aspects, the display 168 can be implemented using any suitable display devices, such as a computer monitor, a touchscreen, a television, etc. In some aspects, the inputs 172 of the secondary computing device 108 may include indicators, sensors, actuatable buttons, a keyboard, a mouse, a graphical user interface, a touch-screen display, etc.

In some aspects, the communication system(s) 176 may include any suitable hardware, firmware, and/or software for communicating with the other systems, over any suitable communication networks. For example, the communication system 176 may include one or more transceivers, one or more communication chips and/or chip sets, etc. In a more particular example, communication system(s) 176 may include hardware, firmware, and/or software that can be used to establish a coaxial connection, a fiber optic connection, an Ethernet connection, a USB connection, a Wi-Fi connection, a Bluetooth connection, a cellular connection, etc. In some aspects, the communication system(s) 176 allows the secondary computing device 108 to communicate with the computing device 104.

In some aspects, the memory 180 may include any suitable storage device or devices that can be used to store instructions, values, etc., that can be used, for example, by the processor 164 to present content using display 168, to communicate with the computing device 104 via communications system(s) 176, etc. The memory 180 may include any suitable volatile memory, non-volatile memory, storage, or any suitable combination thereof. For example, the memory 180 may include RAM, ROM, EEPROM, one or more flash drives, one or more hard disks, one or more solid state drives, one or more optical drives, etc. In some aspects, the memory 180 can have encoded thereon a computer program for controlling operation of secondary computing device 108 (or computing device 104). In such aspects, the processor 164 can execute at least a portion of the computer program to present content (e.g., user interfaces, images, graphics, tables, reports, etc.), receive content from the computing device 104, transmit information to the computing device 104, etc.

The display 116 can be a computer display, a television monitor, a projector, or other suitable displays.

### Exemplary Data Selection and Phenotype Definitions

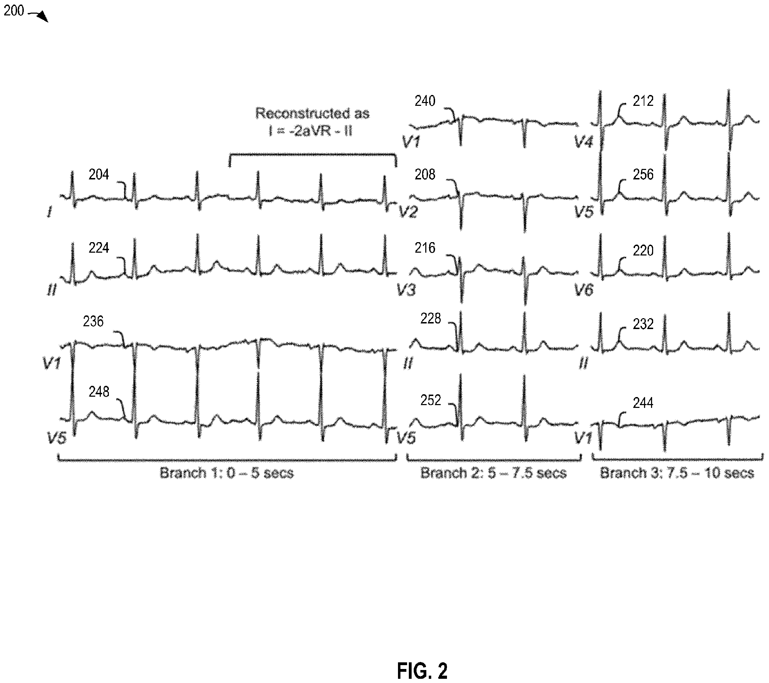

FIG. 2 is an example of raw ECG voltage input data 200. The ECG voltage input data includes three distinct, temporally coherent branches after reducing the data representation from 12 leads to 8 independent leads. Specifically leads aVL, aVF and III may not need to be used because they are linear combinations of other, retained leads. Adding these leads may negatively impact the performance of a model due to overloading of data from certain leads (e.g., duplicate information) and lead to overfitting. In some aspects, these leads may boost model performance when they do not represent duplicate information. Additionally, lead I was computed between the 2.5 and 5 second time interval using Goldberger's equation: -aVR = (I + II) / 2. In some aspects, the data can be acquired at 500Hz. Data not acquired at 500 Hz (such as studies acquired at 250 Hz or 1000Hz) can be resampled to 500 Hz by linear interpolation or downsampling. In some aspects, there may be one branch having leads over a full 10 seconds, 20 seconds, or 60 seconds of one or more leads. In other aspects there may be differing time periods for each branch (e.g., the first branch may include 0-2.5 seconds, the second branch may include 2.5-6 seconds, and the third branch may include 6-10 seconds). In some aspects, the number of branches may match the number of differing periods (e.g., there may be 10 branches each receiving a subsequent 1 second lead sampled at 100Hz, there may be 4 branches each receiving a subsequent 2.5 second lead sampled at 500Hz, etc.). In some aspects, models may be trained and retained for multiple branch, lead, sampling rate, and/or sampling period structures.

As shown, the raw ECG voltage input data 200 can have a predetermined ECG configuration that defines the leads included in the data and a time interval(s) that each lead is sampled, or measured, over. In some aspects, for the raw ECG voltage input data 200, the ECG configuration may include lead I having a time interval of 0-5 seconds, lead V2 having a time interval of 5-7.5 seconds, lead V4 having a time interval of 7.5-10 seconds, lead V3 having a time interval of 5-7.5 seconds, lead V6 having a time interval of 7.5-10 seconds, lead II having a time interval of 0-10 seconds, lead VI having a time interval of 0-10 seconds, and lead V5 having a time interval of 0-10 seconds. The entire ECG voltage input data can have a time interval of 0-10 seconds. Thus, some leads may include data for the entire time interval of the ECG voltage input data, and other leads may only include data for a subset of the time interval of the ECG voltage input data.

In some aspects, the ECG voltage input data 200 can be associated with a time interval (e.g., ten seconds). The ECG voltage input data 200 may include voltage data generated by leads (e.g., lead I, lead V2, lead V4, lead V3, lead V6, lead II, lead VI, and lead V5). In some aspects, the raw ECG voltage input data 200 may include voltage data generated by the leads over the entire time interval. In some aspects, the voltage data from certain leads may only be generated over a portion of the time interval (e.g., the first half of the time interval, the third quarter of the time interval, the fourth quarter of the time interval) depending on what ECG data is available for the patient. In some aspects, a digital image or digital map of a raw ECG voltage input data may be used and each lead identified from the digital image or digital map and a corresponding voltage (e.g., digital voltage data) may be estimated from analysis of the digital image or digital map.

In some aspects, the ECG voltage input data 200 may include first voltage data 204 associated with the lead I and a first portion of the time interval, second voltage data 208 associated with the lead V2 and a second portion of the time interval, third voltage data 212 associated with the lead V4 and a third portion of the time interval, fourth voltage data 216 associated with the lead V3 and the second portion of the time interval, fifth voltage data 220 associated with the lead V6 and the third portion of the time interval, sixth voltage data 224 associated with the lead II and the first portion of the time interval, seventh voltage data 228 associated with the lead II and the second portion of the time interval, eighth voltage data 232 associated with the lead II and the third portion of the time interval, ninth voltage data 236 associated with the lead VI and the first portion of the time interval, tenth voltage data 240 associated with the lead VI and the second portion of the time interval, eleventh voltage data 244 associated with the lead VI and the third portion of the time interval, twelfth voltage data 248 associated with the lead V5 and the first portion of the time interval, thirteenth voltage data 252 associated with the lead V5 and the second portion of the time interval, and fourteenth voltage data 256 associated with the lead V5 and the third portion of the time interval. In this way, the voltage data associated with the portion(s) of the time interval can be provided to the same channel(s) of a trained model in order to estimate risk scores for the patient.

### Exemplary Configurable Single Architecture Machine Learning Aspects

FIG. 3 depicts an exemplary configurable single architecture machine learning model 300, according to some aspects. The configurable single architecture machine learning model 300 may include any number of segments 302. In the depicted example, the configurable single architecture machine learning model 300 includes a segment 302a, a segment 302b and a segment 302c. Further, the configurable single architecture machine learning model 300 may include one or more combined layers 304 (e.g., one or more concatenation layers and/or one or more fully-connected layers ), one or more final output layers 308 having one or more loss functions 310 and modeling output data 312.

It will be appreciated by those of ordinary skill in the art that as used herein, the term "layer" or "layers" may refer to one or more tiers of weights established during the training of one or more machine learning models (e.g., one or more neural networks). In some aspects, modeling approaches may be deployed that do not use explicit weights. For example regression networks and other non-neural network approaches maybe employed that do not involve the training of weights.

Each of the segments 302 corresponds to a respective dissimilar input feature modality. In particular, in the depicted example of the configurable single architecture machine learning model 300, the segment 302a is a customizable imaging/video artificial neural network; the segment 302b is a customizable time series artificial neural network; and the segment 302c is a customizable EHR data artificial neural network. In some aspects, the single architecture machine learning model 300 may include more or fewer segments 302, respectively configurable by the user and/or via automatic means for processing different (or the same) respective input modalities.

The segments 302 may include one or more respective dissimilar input feature modalities 320. For example, as shown in the example of FIG. 3, the segment 302a includes a digital image (e.g., still image and/or video data) input modality 320a; the segment 302b includes a time series (e.g., ECG data) input modality 320b; and the segment 302c includes an EHR data (e.g., HL7 data) input modality 320c. In some aspects, each segment 302 may include different and/or more input modalities. The respective input modalities may be determined via one or more input modality parameters that are provided by the user, selected by the user and/or automatically determined by the present techniques (e.g., by inspecting data input into each respective segment 302).

In some aspects, each of the segments 302 may include one or more respective preprocessing layers 322. For example, as shown in the example of FIG. 3, the segment 302a includes preprocessing layers 322a; the segment 302b includes preprocessing layers 322b; and the segment 302c includes preprocessing layers 322c. In some aspects, the preprocessing layers 322 may be omitted from one or more of the segments 302. The preprocessing layers 322 may perform different respective functions. Generally, the respective preprocessing layers 322 process the respective input modalities 320a, as discussed further below.

In some aspects, each of the segments 302 may include one or more respective sets of isolated layers 324. For example, as shown in the example of FIG. 3, the segment 302a includes one or more sets of isolated layers 324a (e.g., a convolutional neural network (CNN)); the segment 302b includes one or more sets of isolated layers 324b (e.g., a convolutional neural network); and the segment 302c includes one or more sets of isolated layers 324c (e.g., an artificial neural network). The one or more respective sets of isolated layers 324 may be of same or different respective architectures/configurations, in some aspects. For example, in some aspects, the respective convolutional neural networks 324a and 324b of the segments 302a and 302b, respectively, may be of the same architecture, but parameterized differently.

In some aspects, the respective architectures of the convolutional neural networks 324a and 324b may be distinct. In general, the segments operate as independent machine learning processing pipelines, to process the dissimilar input modalities, until their respective outputs are passed to the combined layers 304. One set of isolated layers may be specific to a respective segment and may not interact with any other set of isolated layers specific to different respective segment(s).

As discussed further herein, the one or more combined layers 304 may receive and concatenate (combine) 2a list of inputs (e.g., a list of outputs generated of the respective segments 302). In some aspects, the segments 302 and/or the combined layers 304 may modify the shape of the output tensors of the respective segments 302, so that the combined layers 304 may combine the output tensors into a single tensor having a uniform shape. The combined layer may output the combined single tensor. The combined single tensor may be processed further (e.g., by the one or more fully-connected layers). It will be understood by those of ordinary skill in the art that in some aspects, an explicit combination layer may not be omitted (e.g., the combined layers 304 may be part of another ML model, and thus, not separately depicted).

In some aspects, respective outputs of multiple isolated layers need not match the outputs of one or more other respective isolated layer outputs. For example, the output layers of a sub-neural network specific to dealing with images may be a vector of length 100, but the sub-neural network specific to dealing with tabular features (such as age, sex, vitals), may only be 20. Artificial neural networks may learn that even though the output of the second sub-network is 20, the second sub-network is more important for the modeling task at hand. Another strategy is to ensure an equal weight of multimodal sub-networks and enforce that they all have the same output length. In practice, whether outputs match may be dependent on the design of the overall architecture of the segments 302 towards the modeling task.

In another example, the present techniques may include concatenation of outputs having different dimensional profiles. For example, the combined layers 304 may concatenate a one-dimensional (1-D) (EHR, Genomics, Notes) output, with a two-dimensional (2-D) (ECG, multi-lead) output and a three-dimensional (3-D) (echo, MRI) output. The concatenation layers may flatten these outputs into a tensor of (mini_batch, sum(output_types)). These concatenation layers may be provided as input to a fully-connected network. Post-processing may include one or more steps, such as drop-out, feature regularization and/or max-norm constraints and removal of bias.

In another example, the combination layers 304 may perform tensor fusion. As in the previous example, the outputs of one or more layers may be flattened by the combination layers 304. Here, the flattening may include forcing the outputs to be orthogonal, such that the information contributed by each data modality is forced to be unique through a matrix nuclear norm or similar approach. In some aspects, the combination layers 304 may perform tensor fusion wherein an outer product captures all interactions between modality outputs. In some aspects, the combination layers 304 may perform tensor fusion wherein attention (e.g., self-attention) is be utilized to find one or more predictive areas of a sequence to feed a fully connected network.

In yet another example, the combination layers 304 may perform tensor fusion wherein inputs are padded up to the greatest dimension (e.g., if the most complex output from a data modality is 3 dimensional, then all 1-D and 2-D outputs are reshaped to that dimension using duplication, zero-padding or similar techniques). Thereafter, a secondary convolutional network may be called to analyze this data chunk. This technique may include adding or reducing the weight of each respective modality, depending on how the signal is broadcast across dimensions.

In some aspects, the combination layers 304 may process one or more outputs of respective models as independently produced "features" and provide these features as inputs to a secondary modeling approach and/or to a voting ensemble.

In some aspects, the fully-connected layers may receive as input the output of the combined layers 304. In general, the fully-connected layers perform the function of connecting previous layers to the final output layers. The fully-connected layers may have a number of nodes, each having a respective activation function. Example activation functions include ReLU, Sigmoid, Softmax, etc. In some aspects, an activation function may be omitted. Each of the nodes may correspond to a respective input node of the previous layers, and each node may generate respective outputs. The outputs of the nodes may be provided as inputs to one or more layers, such as the final output layers 308, which may include additional activation functions and/or the loss functions 310. Examples of loss functions include binary cross-entropy, categorical cross-entropy, mean squared error (MSE), L2 loss, etc. Those of ordinary skill in the art will appreciate that any suitable activation functions and loss functions may be used.

The present techniques may include adapting a loss function to work with combined layers. Conventionally, loss functions expect inputs that are compatible and have similar shapes of a similar modality. Such weights are trivially combinable by a loss function. Advantageously, the present techniques enable different modalities to be combined, for example, by normalizing the different modalities using pruning or trimming. In this way, a loss function may be tuned to receive inputs that are expected. In some aspects, multiple layers (e.g., the isolated layers) are tuned to output normalized values, such that once control reaches the combination layers, respective outputs of the isolated layers are compatible and thus, combinable by the combination layers. In this way, the loss function can process the inputs to generate a useful and expected result. The present techniques enable the respective isolated layers to standardize outputs in such a way that such outputs are compatible with other(s) of the isolated layers, at the combination layers. In some aspects, an additional component is used to reconcile outputs of the respective isolated layers. As discussed above, various techniques may be used to combine outputs at the combination layers 304.

The final output of the configurable single architecture machine learning model 300 may be provided as the modeling output 312. This output may vary, depending upon the purpose for which the configurable single architecture machine learning model 300 is being used.

In some aspects, the configurable single architecture machine learning model 300 may be used by the system 100 of FIG. 1A, for example, to train the respective machine learning models 324. For example, the computing device 104 may include computer-executable instructions that, when executed, cause the configurable single architecture machine learning model 300 to be constructed for a particular set of parameters. For example, the user may select three segments (e.g., the three segments 302 of FIG. 3) via the display 116. The computing device 104 may initialize a new architecture (e.g., the configurable single architecture machine learning model 300) and assign each of the segments to the new architecture. For example, the new architecture may be built using an object-oriented programming language (e.g., Python, Java, etc.). The computing device 104 may add preprocessing layers (as optionally selected by the user, and/or based on pre-configuration), one or more ML models (e.g., the ML models 324), one or more concatenation layers, one or more fully connected layers and final output layers with one or more loss function to the new architecture.

In some aspects, the system 100 may independently train one or more of the ML models added to the new architecture. In some aspects, the system may select one or more pre-trained ML models (e.g., the convolutional neural network 324a may be trained using the training data database 124, while the convolutional neural network 324b is loaded from the trained models database 128).

The computing device 104 may then operate the new architecture, including the trained models therein, for example using information received via the communication network (e.g., information included in the ECG database 120, information received directly from an ECG machine, EHR information, imaging information, etc.). The received information may be provided to the appropriate segment of the new architecture (e.g., the respective segments 302) based on the source of the information. The computing device 104 may receive the modeling output 312, and may process the output further. For example, the computing device 104 may transmit the output 312, store the output 312, perform a computation using the output 312, etc. Additional examples of parameterizing, constructing, training and operating the architecture 300 are discussed below.

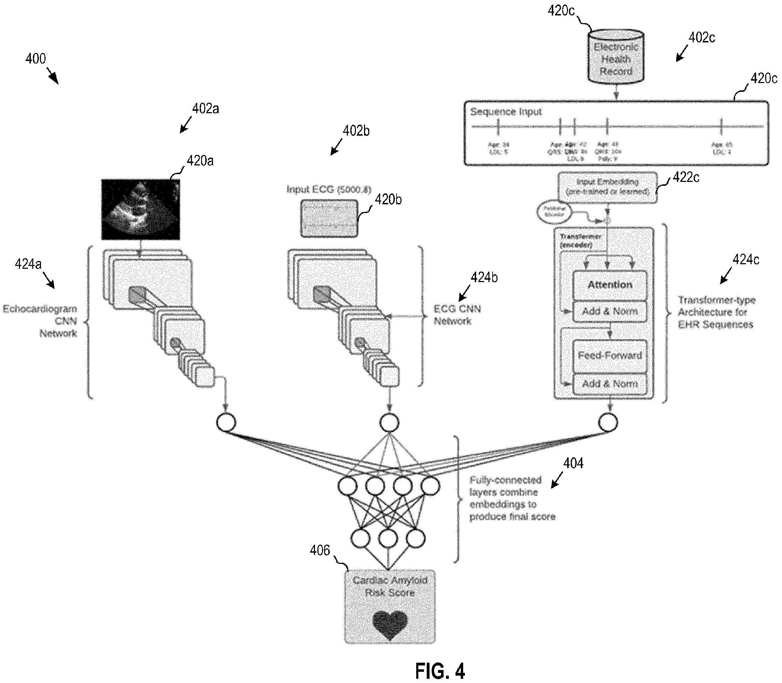

Turning to FIG. 4, an exemplary configurable single architecture machine learning model 400 for generating a cardiac amyloid risk score is depicted, according to some aspects. The single architecture machine learning model 400 may include segments 402a, 402b and 402c that correspond, respectively, to a customizable imaging/video artificial neural network, a customizable time series artificial neural network, and a customizable EHR data artificial neural network. For example, the segments 402a-402c may correspond, respectively, to the segments 302a-302c of FIG. 3. The configurable single architecture machine learning model 400 may further include one or more fully-connected layers 404 and a modeling output 406. The one or more fully-connected layers 404 and the modeling output 406 may correspond, respectively to the combined/ fully-connected layers of FIG.3 and the modeling output 312 of FIG., in some aspects. Further, the fully-connected layers 404 may include a concatenation layer (not depicted) that may correspond to the combined layers 304, in some aspects.

Further, the segments 402a-402c each include respective input modalities 420a-420c. The respective input modalities may correspond to the input modalities 320a-310c, respectively, in some aspects. For example, the input modality 420a may be an image/video data input modality; the input modality 420b may be a time series input modality (e.g., an ECG input modality having a shape of (5000,8)); and the input modality 420c may include one or more EHR sequence inputs including patient age and other demographic/diagnostic information.

Regarding preprocessing, in the depicted example, the segments 402a and 402b may lack any preprocessing layers. In contrast, the segment 402c may include one or more preprocessing layers 422c that perform input embedding. The input embedding may include either pre-trained or learned pre-processing, in some aspects. The preprocessing layers 422c may include positional encoding, in some aspects.

The input modalities 420a-420c may be input into respective sets of isolated layers 424a-424c, respectively, across the segments 402a-402c. For example, as depicted, the isolated layers 424a of the segment 402a may be an echo convolutional neural network model that is configured to process video/image data modalities 420a. The set of isolated layers 424b may be an ECG convolutional neural network model that is configured to process time series input modalities 420b. The set of isolated layers 424c may be a transformer machine learning model configured to process the EHR input modalities 420c. For example, the transformer model 424c may include an encoder mechanism, an attention mechanism, and a feed-forward artificial neural network. Those of ordinary skill in the art will appreciate that many other model configurations/types are available for each of the ML models 424. The output of the models may be combined by the fully-connected layers 404 to generate the final modeling output 406; in this example, a cardiac amyloid risk score.

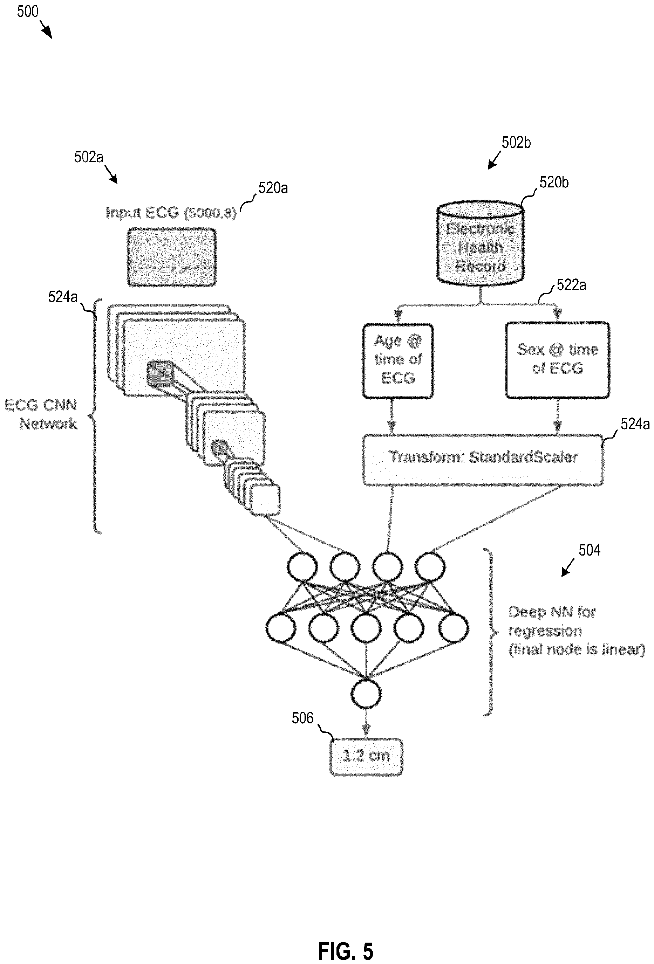

FIG. 5 depicts an exemplary loss function 500 tailored for regression, according to some aspects. The loss function 500 may include one or more segments 502 corresponding to two input modalities 520. The input modality 520a may correspond to a time series, such as the time series input modality 420b of FIG. 4. FIG. 5 includes an example of numerical feature processing at one or more preprocessing layers 522a. Specifically, the input modality 520b includes one or more preprocessors 522a, that split EHR data into two respective numerical features and provide the split data into a transformer set of isolated layers 524a. The respective outputs of the segment 502a and the segment 502b may be concatenated and/or processed further by a deep artificial neural network machine learning model 504 to perform regression, and to output a final value 506. For example, in some aspects, the final layer of the artificial neural network set of isolated layers 504 may be a linear activation function. For example, in the depicted example the final value 506 may represent a septal wall thickness. Many different configurations, using different input modalities, ML models and/or final output layers are envisioned. For example, in some aspects, the concatenation or combination process may include determining whether dimensions of the combined isolated layers 504 match along the axis of concatenation. For instance, if the output dimension of an ECG modality sub network is a 1D array, then the other outputs from all other modalities must also be a 1D array or be modified as such.

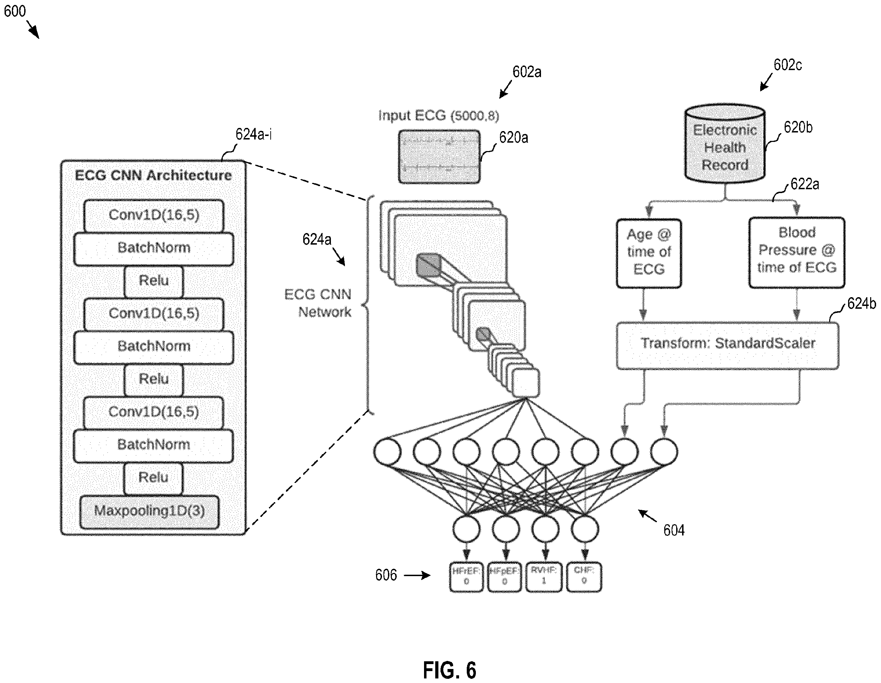

FIG. 6 depicts an exemplary deep learning network architecture 600 for multi-class classification, according to some aspects. The deep learning network architecture 600 may include one or more respective segments 602 corresponding to two input modalities 620. The input modality 620a may correspond to a time series, such as the time series input modality 420b of FIG. 4 and/or the time series input modality 520a of FIG. 5, in some aspects. The input modality 620b may correspond to the input modality 520b, in some aspects. The deep learning network architecture 600 may include respective sets of isolated layers 624a and 624b. While the segment 602c includes preprocessing layers 622a, the segment 602a lacks any preprocessing layers between the input modality 620a and the ML model 624a.

FIG. 6 further depicts an architecture detail block 624a-i, corresponding to the architecture of the set of isolated layers 624a, which in the depicted example is an ECG CNN architecture. The architecture detail block 624a-i depicts a CNN architecture having a 1D convolutional layer having 16 input channels and 5 output channels, a batch normalization preprocessing layer, ReLU activation function, followed by additional convolution, batch normalization and activation layers, and finally, a max pooling layer. It will be appreciated by those of ordinary skill in the art that the architecture detail block 624a-i is for exemplary purposes only, and that many other configurations are envisioned, both of CNNs and other modeling types.

FIG. 6 include another example of numerical feature processing as discussed above. For example, FIG. 6 includes preprocessors 622a that split EHR data into two (or more) or respective numerical features and provide the split data (respective features) into the transformer set of isolated layers 624b. Whereas FIG. 5 depicted the numerical features of the patient's age at the time of an ECG, and the patient's sex at the time of an ECG, FIG. 6 depicts the features of the patient's age at the time of an ECG and the patient's blood pressure at the time of an ECG. It should be appreciated that many more features are envisioned, both numerical and categorical, as well as feature censoring and other feature-based techniques.

The respective outputs of the segment 602a and the segment 602b may be concatenated and/or processed further by one or more combination layers 604 to perform multi-class classification, and to output a plurality of classification output values 606. In some aspects, the output values may represent respective probabilities from 0.0 to 1.0 (inclusive) corresponding to respective heart failure classifications at each of the respective classification output values 606.

In some aspects, the present techniques may be recursive/nested. For example, the segment 602a of FIG. 6 may include the entirety of the loss function 500 of FIG. 5.

### Exemplary Computer-Implemented Methods

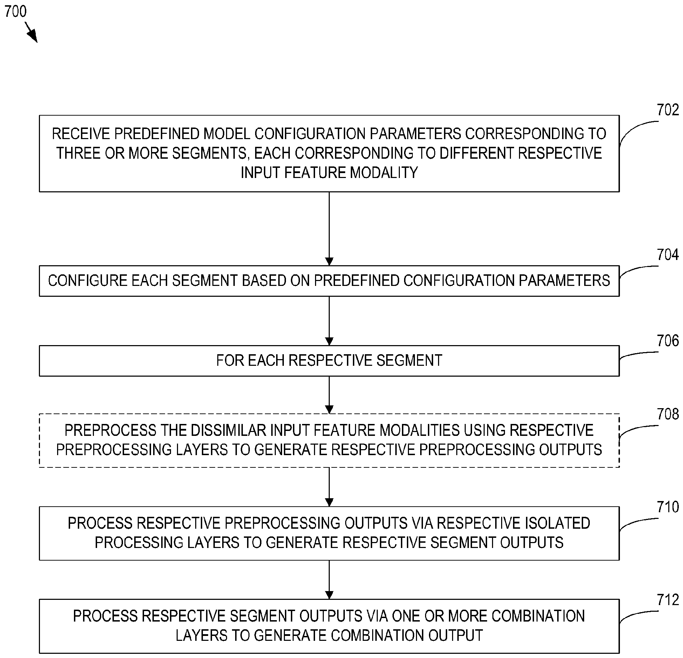

FIG. 7 depicts an exemplary computer-implemented method 700, according to some aspects. The method 700 may be performed by the architecture 300 of FIG. 3, in some aspects. The architecture 400 of FIG. 4, the loss function 500 of FIG. 5 and/or the architecture 600 of FIG. 6 may also be used, respectively, to perform the method 700, in aspects. Further, those of ordinary skill in the art will appreciate that the method 700 may be executed by yet further architectures that while not depicted, are envisioned using the principles described by present techniques.

The method 700 may include receiving predefined model configuration parameters corresponding to three or more model segments, each respective model segment corresponding to a different respective input feature modality, each respective model segment including one or more respective preprocessing layers, and each respective model segment including one or more respective set of isolated processing layers (block 702). For example, the model configuration parameters may be received from the inputs 152 of the computing device 140 and/or the inputs 172 of the computing device 108. In some embodiments, the model configuration parameters may be received via the network 112. The predefined model configuration parameters may be received via the ECG analysis application 132. In some aspects, the model configuration parameters may be entered and/or selected by a user via a hardware interface (e.g., via the input 152 of the computing device 104 of FIG. 1B).

For example, the method 700 may receive parameters selected via a touch screen of the display 116 of FIG. 1A, via the display 116 of FIG. 1B, etc. The predefined model configuration parameters may be represented as a nested data structure, such as XML, JSON, etc. The nested data structure may include parameters defining one or more respective input modalities (e.g., a customizable imaging/video neural network, a customizable time series neural network, a customizable EHR data neural network etc.). The nested configuration data may further include an indication of one or more preprocessing layers, such as the preprocessing 322a-c of FIG. 3.

The method 700 may include configuring each model segment based on the predefined model configuration parameters (block 704). Generally, the method 700 may execute computer-executable instructions that create/load and configure one or more single architecture machine learning model (e.g., the single architecture machine learning model 400 of FIG. 4). In particular, instructions included in one or more computing devices (e.g., the computing device 104) may set the number and characteristics of one or more segments of an ML model (e.g., the segments 402 of the single architecture machine learning model 400 of FIG. 4). The method 700 may further include configuring one or more trained machine learning models (e.g., via loading one or more models via the training database 124).

The method 700 may include configuring preprocessing instructions with respect to one or more segments of the ML model (e.g., the preprocessing layers 322 of FIG. 3). For example, the method 700 may include, for each respective model segment: preprocessing the dissimilar input feature modalities using the respective preprocessing layers to generate respective preprocessing outputs (block 706). For example, with respect to FIG. 3, the method 700 may include the segments 302 applying one or more respective preprocessing steps 322 to the respective input modalities 320.

Further, it should be understood that preprocessing may occur at any stage within a given architecture, including at the beginning of any segment, as a final layer and/or anywhere in between.

As shown in FIG. 4, dissimilar features may include ECG features and/or EHR features, and modeling outputs may relate to patient risk. For example, in some aspects, the dissimilar input feature modalities may electrocardiogram features and electronic health record features; and the modeling output is a probability from 0 and 1 representing a future risk to a patient. In some aspects, the dissimilar input feature modalities may electrocardiogram features, echocardiogram features and electronic health record features; and the modeling output may be a probability from 0 and 1 representing a future risk to a patient. In some aspects, the dissimilar input feature modalities may include retinal scan image features and electronic healthcare record features; and the modeling output may be a probability from 0 and 1 representing a patient's future risk of onset diabetes.

Those of ordinary skill in the art will appreciate that one of the advantageous aspects of method 700 is that a number of the steps may be performed in parallel by the architectures discussed herein. For example, with reference to FIG. 3, each of the segments 302 may correspond to a respective set of computer-executable instructions, and the processor 144 of FIG. 1B may include a number of cores, each of which executes one of the respective sets of instructions. In this way, the segments 302 perform their respective processing work in parallel, leading to faster execution of the overall modeling in the architecture 300. In some aspects, the processor 144 may include multiple processors (e.g., a compute cluster of ten or more GPUs). The method 700 may use one or more of the multiple processors to execute the respective sets of instructions corresponding to the segments 302.

In some aspects, the method 700 may include processing the respective preprocessing outputs via the respective isolated processing layers to generate respective segment outputs (block 708). For example, the preprocessing outputs may correspond to the outputs of the preprocessing layers 322a. The segment outputs may correspond to the respective outputs of the ML models 324 of FIG. 3. Because preprocessing is optional/not required in all circumstances, block 708 is shown with dashed borders.

The method 700 may include processing the respective segment outputs via one or more combination layers to generate a combination output (block 710). The method may include processing the combination output via one or more fully-connected layers to generate fully-connected output (block 712). For example, in some aspects, the processing of the respective segment outputs to generate the combination may correspond to the processing performed by the combined layers 304 of FIG. 3, and the processing via one or more fully-connected layers may correspond to the processing performed by the fully-connected layers.

The method 700 may include processing the fully-connected output via one or more final output layers to generate modeling output, wherein the one or more final output layers implements one or more loss functions, and wherein the modeling output has an output type. Again, with reference to FIG. 3, the processing of the fully connected output via the one or more final output layers may correspond to the processing of the final output layers 308 of FIG. 3. In some aspects, the one or more final output layers implement one or more loss functions. In some aspects, the modeling output has an output type.

The method 700 may include normalizing the outputs of each of the respective sets of isolated processing layers in the one or more segments. In this way, the output of those layers can be combined and joined together for processing by loss function(s0 in the combination layers.

The one or more loss functions may include i) a regression function; ii) a binary classification function; iii) a multi-class classification function; and/or iv) a survival modeling function. In some cases, the regression function is a linear activation function included in one or more neurons of a dense output layer. Further, in some aspects, the regression function may include i) a mean squared error loss function; ii) a mean squared logarithmic error loss function; iii) a mean absolute error loss function; and/or iv) a Huber loss function.

In some aspects, the binary classification function may be a sigmoid activation function included in one or more neurons of a dense output layer. The binary classification function may include: i) a binary cross entropy loss function; ii) a Kullback-Leibler divergence loss function; iii) a Hinge function loss function; iv) a squared Hinge loss function; and/or v) a focal loss function.

The method 700 may be used to perform multi-class classification, as depicted for example in FIG. 6. In some aspects, the multi-class classification function may be a softmax activation function included in one or more neurons of a dense output layer. The multi-class classification function may include i) a categorical cross entropy loss function; ii) Kullback-Leibler divergence loss function; iii) a sparse multiclass cross-entropy loss function; iv) a focal loss function; and/or v) a negative log-likelihood function. In some aspects, the machine learning models may include a multiclass classification model; the loss functions may include a multiclass classification loss function; and the final output layers may include a multiclass classification output layer.

The method 700 may include survival modeling. The survival modeling function may be a linear activation function included in one or more neurons of a dense output layer. The survival modeling function may include a Cox-proportional hazard loss function. For example, in some aspects, the machine learning models may include a survival modeling model; wherein the loss functions may include a survival loss function; and the final output layers may include a survival output layer. In some aspects, the dissimilar input feature modalities include electrocardiogram features and electronic healthcare record features; and the modeling output may be a numerical value indicating a number of days until an event as a survival curve. In still further aspects, the dissimilar input feature modalities may include genomic data features, echocardiogram features, electrocardiogram features and electronic healthcare record features; and the modeling output may be a numerical value indicating a number of days until an event as a survival curve.

In some aspects, the machine learning models may include a binary classification model, the loss functions may include a binary classification loss function, and/or the final output layers (e.g., the final output layers 308 of FIG. 3) include a binary classification output layer. In some aspects, the dissimilar input feature modalities include electrocardiogram features and electronic health record features; and the modeling output is a probability from 0 and 1 representing a future risk to a patient.

In some aspects, the machine learning models of method 700 may include at least one of i) an artificial neural network; ii) a convolutional neural network; or iii) a sequence neural network. For example, as shown in FIG. 3, the ML model 324c is an artificial neural network. One or more ML models using ML modeling techniques now known or later developed may be substituted for one or more of the ML models 324, in aspects.

In the method 700, receiving predefined model configuration parameters may include receiving one or more of any of the following i) a digital image or a digital video input; ii) a time series input; iii) an electronic health records data input; iv) an electrocardiogram input; v) an echocardiogram input; vi) a computed tomography input; vii) a magnetic resonance imaging input; viii) a tabular data input; or ix) a natural language string input.

As discussed, in the method 700, the dissimilar input feature modalities may include ECG time series data. In such cases, preprocessing the plurality of dissimilar input feature modalities via respective preprocessing layers to generate the respective preprocessing outputs includes preprocessing at least one of the model inputs via i) an electrocardiogram lead recalibration; ii) an electrocardiogram lead amplitude standardization; iii) an electrocardiogram lead augmentation; iv) an electrocardiogram data restructuring; v) an electrocardiogram lead rescaling; or vi) a Fourier space transformation.

As discussed above, the method 700 may include preprocessing the model inputs via one or more respective preprocessing layers to generate the respective preprocessing outputs, including preprocessing at least one of the model inputs via categorical feature processing; ii) numerical feature processing; or iii) feature censoring. Such techniques may be used when at least one of the model inputs includes tabular data, and/or for other input types.

As described with respect to the architectures above, in some aspects, the method 700 may include receiving image and/or video data. In such cases, preprocessing the model inputs via the one or more respective preprocessing layers to generate the respective preprocessing outputs may include preprocessing at least one of the model inputs via: i) image size normalization; ii) image augmentation; or iii) a Fourier space transformation. It is envisioned that other image-based techniques may be applicable, in some circumstances.

As discussed above, preprocessing is optional, both with respect to overall architectures and each segment within a given architecture. While it is true that in reality, almost all datasets need at least some cleaning performed to place them in the best possible condition for modeling, it is also true that in some aspects, preprocessing may not need to be performed because the data is already in a clean format.

Further, in some aspects, data is examined for discrepancies against an expected format and preprocessed to match the format if any are identified. For example, in some aspects, the method 700 includes receiving an expected format; and preprocessing the model inputs via the one or more respective preprocessing layers to generate the respective preprocessing outputs based on the expected format.

Further, formatting may be parameterized, in some aspects. For example, using patient age as an example, a downstream recipient may desire raw age, whereas another recipient desires to receive age in buckets (e.g., 40-50, 50-60, etc.). Yet another recipient may desire to see age as a Boolean value representing whether the patient age exceeds a given value (e.g., 65 years). For example, one or more parameters describing expected format(s) may be included in the predefined model configuration parameters at block 702 of FIG. 7. As noted, such parameters may be stored preferences, and/or set *ad hoc* by a user. The expected formats may include, for example, single numeric values, numeric ranges and/or Boolean values, as well as any other suitable data formats.

The method 700 may include using a deep network for regression, as shown in FIG. 5. For example, in some aspects, the machine learning models may include a binary classification model; the loss functions may include a regression loss function; and the final output layers may include a regression output layer. In some aspects, the dissimilar input feature modalities may include electrocardiogram features and electronic healthcare record features; and the modeling output may be a numerical value indicating a physiological aspect of a patient.

The method 700 may be used to classify patients as having heart failure. In aspects, the dissimilar input feature modalities include electrocardiogram features and electronic healthcare record features; and the modeling output may be a respective probability from 0 to 1 corresponding to a plurality of heart failure classifications.

In some aspects, the plurality of dissimilar input feature modalities includes a structured data set and a time series data set, and preprocessing the dissimilar input feature modalities using the respective preprocessing layers to generate respective preprocessing outputs at block 708 of the method 700 may include preprocessing the structured data set using at least one of (i) categorical feature processing, (ii) numerical feature processing, or (iii) feature censoring. The structured data set may be a tabular data set, in some aspects.

Further, the preprocessing at block 708 of the method 700 may further include preprocessing the dissimilar input feature modalities using the respective preprocessing layers to generate respective preprocessing outputs includes preprocessing the time series data set using at least one of (i) electrocardiogram lead recalibration, (ii) electrocardiogram lead amplitude standardization, (iii) electrocardiogram lead augmentation, (iv) electrocardiogram data restructuring, (v) electrocardiogram lead rescaling, or (vi) Fourier transformation. The one or more loss functions include a binary classification loss function that generates one of (i) a positive prediction, or (ii) a negative prediction.

### Exemplary Training

The computing device 104 may train the ML models within the architecture 300, the architecture 400, the architecture 500 the architecture 600 (for example) to perform multimodal modeling using inputs including ECG features, EHR and/or genomic features, and/or imaging features. Specifically, the present techniques may support EHR features ingestion, preprocessing and feeding these features alongside processed ECG features in a single neural network for cardi disease modeling. The system 100 may train the respective models of the architectures discussed herein by bucketing features into one or more feature types (e.g., time series, structured, and/or imaging features). The user may define such feature types using the configuration parameters discussed with respect to FIG. 1A. In some aspects, the features may be automatically detected based on the inputs. For example, the system 100 may detect certain feature types.

The feature types may include time series features. Time series feature preprocessing and data feeding may be handled via configuration files for full flexibility for time series modeling. The feature types may include structured features. Structured features may include categorical features (e.g., "male / female", "inpatient / outpatient / ER", etc.). Structured features may include numerical features (e.g., 0.35 ejection fraction, 120 Vitals BP, etc.) Structured features may include alternative time series (ex. EEG, sensor data, etc.). Structured features may include Boolean features (e.g., True, False). Structured features may include an ignore preprocessing type, a predefined user column that requires no preprocessing. The feature types may include imaging features.

Preprocessing may include batched normalization, such as scaling ECG waveforms to smaller numbers. In some aspects, preprocessing of individual feature inputs may be configured based on three options (skip, numerical and categorical). In the skip option, the system 100 may allow the trained models to ingest a value (e.g., a CHA2DS2-VASc) value as-is (e.g., because the value does not need to be preprocessed or has already been preprocessed upstream). In the numerical option, the system 100 may normalize numerical values to a predetermined distribution. In the categorical option, the system 100 may encode string values and categories into numbers.

Similar to time series preprocessing and data feeding, imaging features may be a third feature type input into a single neural network model. Bucketed features may be automatically numerically scaled and/or feature scaled according to user-specific parameters. In some aspects, post-processed and scaled features are fed into specific neural network layers that determine all of the complex non-linear interactions of each feature set. These layers may be completely configurable to allow for highly customizable layers towards a variety of multiple clinical feature sets. For example, a custom 1D Convolution Neural net can be used to process time series features, a fully-connected neural network can be used to process structured datasets, such as EHR and/or genomic data, and a 2D/3D convolutional neural network can be used to process imaging feature sets. For instance, the present multi-modal techniques may include processing genomics data, including inputs as large as whole genome sequencing (WGS) to more targeted panels such as an assay for tumors (such as a 596 gene panel). These types of inputs may be analyzed by a variety of methods including deep learning (e.g., CNNs, RNNs, transformers) and machine learning (e.g., SVM, gradient boosted models, logistic regression) algorithms. These outputs may be 1-dimensional. These non-linear interaction outputs from each feature set type may be merged into a single encompassing neural network architecture.

The system 100 may combine features according to different concatenation strategies. In one strategy, a temporal co-variant data set (e.g., an ECG time series) may be combined with an echocardiogram video having multiple images per frame or frames/second. The system 100 may concatenate and layer the ECG time series data and the echocardiogram frames. In some aspects, a strategy may combine EHR features with ECG or echo data by aggregating one EHR feature with multiple ECG data points. In some aspects, a strategy may combine ECG data with summarized data (e.g., a median, average, maximum or minimum value) across time (e.g., across several weeks). In yet another strategy, the system 100 may generate a table, with each row corresponding to points in time, each row including a respective ECG, echo and lab value.

Via the configuration and merging of multimodal/heterogeneous input types discussed above, the present techniques overcome the drawbacks discussed above by natively preprocessing and incorporating clinical and/or tabular features alongside ECG features within a single neural network model towards disease predictions, while providing native support for categorical, Boolean, and numerical features; and doing so in a disease agnostic and modeling endpoint agnostic fashion. The present techniques are also supported for local and/or cloud based developments and deployments.

The trained models may be used in the cardiology space for clinical usage alongside licensing usages by partners. For example, the architecture 400 of FIG. 4 may utilize time series features (e.g., ECGs), structured EHR features, imaging features (e.g., echocardiograms), and / or genomic features to help predict the future risk of various cardiac event onsets. The present techniques enable all current and future cardio Al work to fully utilize multimodal feature sets into a single neural network model that can be trained for classification, time-to-event, regression, and alternate modeling schemas.

The present techniques may be used to retrofit existing models. For example, an existing model may only consider a limited number of demographic features (e.g., patient age / sex). The system 100 may load the existing model and utilize this invention to reconfigure the model to include information beyond demographic features. The present techniques may also determine model interpretability and feature importance. For example, by extracting the embedding space features and associated weights for each feature set, the system 100 may rank input features based on most to least important towards a disease prediction. For instance, the system 100 may determine that the most important features for a high-risk prediction for an AF patient are the ECG features, the patient age, and the ejection fraction. The least important features may be the patient sex, the patient tropinin lab values, and the patient smoking history. A further example of retrofitting existing models is provided below.

The model 300 may be a comprehensive multimodal model that may leverage additional EHR features to broaden the predictive disease subspace and significantly boost model performance beyond unimodal feature sets. The model 300 may be a robust model that utilizes more than ECG, patient age, and patient sex as features. Thus, the present techniques may benefit patients by correctly flagging patient risk for events months to years prior to their actual event. Further, the present techniques may be used to create robust multimodal risk models for patient screening and patient selection for pharma companies.

The architectures discussed herein may be configured to receive a plurality of input data types, each having a respective data shape and respective data features.

For example, a given model may be configured to receive an input data type that has a particular data shape (e.g., a size of 5 frames each having 5000 pixels) and particular data features (e.g., ECG time series data features). The model may be configured to interpret the first input data type using one or more time series neural network layers included within the model.

The model may be configured to receive an input data type. The input data type may have a particular data shape (e.g., 3 frames each having height and width of 256 pixels x 256 pixels, respectively) and particular data features (e.g., echocardiogram image features). The model may be configured to interpret the input data type using one or more imaging features neural network layers.

The model may be configured to receive an input data type having a particular data shape (e.g., a structured data type) and particular data features (e.g., column-labeled tabular data including EHR and/or genomic data features). The model may be configured to interpret the input data type using one or more structure feature neural network layers. Unlike the tabular data of Vaid et al., the present modeling techniques allows for full flexibility for users to customize preprocessing and layer definitions to ensure robust and high-quality models.

In some aspects, the model may be configured to receive more or fewer input data types having different particular data shapes and/or different particular data features. The configuration may be performed by the system 100, as discussed above.

As discussed above, the model may include a concatenation operation that combines multiple input data types into a set of fully-connected layers. The fully-connected layers may be configured to process the input data types to generate a prediction as to a cardiac state (e.g., as depicted, the probability of HF within one year).

As discussed above, the present techniques may be used to configure the model to have different architecture based on configuration parameters. Further examples of different model types are depicted below. Examples of cardio condition modeling that may be performed using the model are as follows:

| | |
|---|---|
| FFR | CNN for identifying FFR abnormalities from ECG which may also be applied to identify cardiac events. |
| | One or more CNNs for each composite model from ECG, Age, Sex, one CNN for each cardiac event, previously filed |
| AS | Aortic Stenosis prediction (1 year future incidence or active disease) from ECG, Age, Sex, uses same CNN as previous filings |
| MS | Prediction of mitral stenosis (1 year future incidence or active disease) |
| AR | Prediction of aortic regurgitation (1 year future incidence or active disease) |
| MR | Prediction of mitral regurgitation (1 year future incidence or active disease) |
| TR | Prediction of tricuspid regurgitation (1 year future incidence or active disease) |
| EF | Prediction of reduced left ventricular ejection fraction (EF) (1 year future incidence or active disease) |
| IVS | Prediction of reduced increased interventricular septal (IVS) thickness (1 year future incidence or active disease) |
| CA | Cardiac Amyloidosis prediction, uses unknown machine learning model (not CNN); one feature is interventricular septal thickness (IVSD) determined from ECG |
| Stroke | Stroke prediction from ECG, Age, Sex, uses same CNN as previous filings |
| | Stroke prediction from customized phenotypical categorization to identify negative occurrence, active occurrence, any future occurrence of stroke within one or more time periods. |
| | Customize phenotypes from ICD codes, symptoms, medications, stroke registry flags (incidents) |
| Long QTc | CNN for prediction of future long QTc |
| Stroke w/ Time Series | Stroke prediction from identified features which are captured multiple times within the dataset and building a time series representation from them, providing the time series representation along with EHR features of comorbidities, symptoms, and encounter dates to a CNN model |

The input data structure to the model may include a first including leads I, II, V1, and V5, acquired from time (t) = 0 (start of data acquisition) to t=5 seconds (e.g., the first voltage data, the sixth voltage data, the ninth voltage data, and the twelfth voltage data); a second branch including leads V1, V2, V3, II, and V5 from t=5 to t=7.5 seconds (e.g., the second voltage data, the fourth voltage data, the seventh voltage data, the tenth voltage data, and the thirteenth voltage data); and a third branch including leads V4, V5, V6, II, and V1 from t=7.5 to t=10 seconds (e.g., the third voltage data, the fifth voltage data, the eighth voltage data, the eleventh voltage data, and the fourteenth voltage data) as shown in FIG. 2. The arrangement of the branches can be designed to account for concurrent morphology changes throughout the standard clinical acquisition due to arrhythmias and/or premature beats. For example, the model may need to synchronize which voltage information or data is acquired at the same point in time in order to understand the data. Because the ECG leads are not all acquired at the same time, the leads may be aligned to demonstrate to the neural network model which data was collected at the same time. It is noted that not every lead needs to have voltage data spanning the entire time interval. This is an advantage of the model, as some ECGs do not include data for all leads over the entire time interval. For example, the model may include ten branches, and can be trained to generate a risk score based in response to receiving voltage data spanning subsequent one second periods from ten different leads. As another example, the model may include four branches, and can be trained to generate a risk score based in response to receiving voltage data spanning subsequent 2.5 second periods from four different leads. Certain organizations such as hospitals may use a standardized ECG configuration (e.g., voltage data spanning subsequent one second periods from ten different leads). The model may include an appropriate number of branches and be trained to generate a risk score for the standardized ECG configuration. Thus, the model 40 can be tailored to whatever ECG configuration is used by a given organization.

As discussed, in some aspects, the model may include a convolutional component, inception blocks, and a fully-connected dense layer component. These components may be enabled or disabled, and their various properties defined, depending on the configuration parameters received by the system 100. In some aspects, the configuration parameters may be default parameters.

The convolutional component may start with an input for each branch followed by a convolutional block. Each convolutional block included in the convolutional component 400A may include a 1D convolutional layer, ReLU activation function, and a batchnorm layer, in series.

Next, this convolutional block can be followed by four inception blocks in series, where each inception block may include three 1D convolutional blocks concatenated across the channel axis with decreasing filter window sizes. Each of the four inception blocks can be connected to a 1D max pooling layer, where they are connected to another single 1D convolutional block and a final global averaging pool layer. The outputs for all three branches can be concatenated and fully-connected to the dense layer component. The dense layer component may include four dense layers of 256, 64, 8 and 1 unit(s) with a sigmoid function as the final layer.

All layers in the architecture can enforce kernel constraints and may not include bias terms. In some aspects, the AdaGrad optimizer can be used with a learning rate of 1e⁻⁴ 45, a linear learning rate decay of 1/10 prior to early stopping for efficient model convergence, and batch size of 2048. In some aspects, the model can be implemented using Keras with a TensorFlow backend in python and default training parameters were used except where specified. In some aspects, AdaGrad optimizer can be used with a learning rate of 1e^{-4 45}, a linear learning rate decay of 1/10 prior to early stopping for efficient model convergence at patience of three epochs, and batch size of 2048. In some aspects, differing model frameworks, hypertuning parameters, and/or programming languages may be implemented. The patience for early stopping was set to 9 epochs. In some aspects, the model 400 can be trained using NVIDIA DGX1 and DGX2 machines with eight and sixteen V100 GPUs and 32 GB of RAM per GPU, respectively.

In some aspects, the model can additionally receive electronic health record data points such as demographic data, which may include age and sex/gender as input features to the network, where sex can be encoded into binary values for both male and female, and age can be cast as a continuous numerical value corresponding to the date of acquisition for each 12-lead resting state ECG. In some aspects, other representations may be used, such as an age grouping 0-9 years, 10-19 years, 20-29 years, or other grouping sizes. In some aspects, other demographic data such as race, smoking status, height, and/or weight may be included. In some aspects, the EHR data points may include laboratory values, echo measurements, ICD codes, and/or care gaps. The EHR data points (e.g., demographic data, laboratory values, etc.) can be provided to the model at a common location.

The EHR data points (e.g., age and sex) can be fed into a 64-unit hidden layer and concatenated with the other branches. In some instances, these EHR features can be extracted directly from the standard 12-lead ECG report. In some aspects, the model can generate ECG information based on voltage data from the first branch, the second branch, and the third branch. In some aspects, the model can generate demographic information based on the demographic data. In some aspects, the demographic information can be generated by inputting age and sex were input into a 64-unit hidden layer. The demographic information can be concatenated with the ECG information, and the model can generate a risk score based on the demographic information and the ECG information. Concatenating the ECG information with the separately generated demographic information can allow the model to individually disseminate the voltage data from the first branch, the second branch, and the third branch, as well as the demographic data, which may improve performance over other models that provide the voltage data and the demographic data to the model at the same channel.

In some aspects, the risk score can be indicative of a likelihood the patient will suffer from a condition within a predetermined period of time from when electrocardiogram data (e.g., the voltage data from the leads) was generated. In some aspects, the condition can be AF, mortality, ST-Elevation Myocardial Infarction (STEMI), Acute coronary syndrome (ACS), stroke, or other conditions indicated herein. In some aspects, the model can be trained to predict the risk of a patient developing AF in a predetermined time period following the acquisition of an ECG based on the ECG. In some aspects, the time period can range from one day to thirty years. For example, the time period may be one day, three months, six months, one year, five years, ten years, and/or thirty years.

In some aspects, Bayesian-based layers may be included in the model. These layers may deliver non-deterministic results alongside or instead of deterministic results such as numeric and/or Boolean outputs. This may represent a further advantageous improvement over conventional modeling techniques, which output deterministic answers even in the presence of faulty inputs (e.g., noisy/missing ECGs, artifacts in EHR (e.g., missing sex, negative age, smoking history blank, etc.), speckle or noise in images, etc.). By including Bayesian layers, the model may be trained to include a confidence interval along with a deterministic prediction, which may be used to accord weight to the prediction of the model.

In some aspects, the ML models can receive ECG voltage data generated over a single time interval.

In some aspects, the model can be a deep neural network. In some aspects, the model may include a single branch that can receive ECG voltage input data generated over a single time interval (e.g., ten seconds). As shown, the model can receive ECG voltage input data generated over a time interval of ten seconds using eight leads. In some aspects, the ECG voltage input data may include five thousand data points collected over a period of 10 seconds and 8 leads including leads I, II, V1, V2, V3, V4, V5, and V6. The number of data points can vary based on the sampling rate used to sample the leads (e.g., a sampling rate of five hundred Hz will result in five thousand data points over a time period of ten seconds). The ECG voltage input data 428 can be transformed into ECG waveforms.

As described above, in some aspects, the ECG voltage input data can be "complete" and contain voltage data from each lead (e.g., lead I, lead V2, lead V4, lead V3, lead V6, lead II, lead VI, and lead V5) generated over the entire time interval. Thus, in some aspects, the predetermined ECG configuration may include lead I, lead V2, lead V4, lead V3, lead V6, lead II, lead VI, and lead V5 having time intervals of 0-10 seconds. The model can be trained using training data having the predetermined ECG configuration including lead I, lead V2, lead V4, lead V3, lead V6, lead II, lead VI, and lead V5 having time intervals of 0-10 seconds. When all leads share the same time intervals, the model can receive the ECG voltage input data at a single input branch 432. Otherwise, the model may include a branch for each unique time interval may be used as described above.

The ECG waveform data for each ECG lead may be provided to a 1D convolutional block where the layer definition parameters (n, f, s) refer, respectively, to the number of data points input presented to the block, the number of filters used, and the filter size/window. In some aspects, the number of data points input presented to the block can be five thousand, the number of filters used can be thirty-two, and the filter size/window can be eighty. The 1D convolutional block can generate and output a downsampled version of the inputted ECG waveform data to the inception block. In some aspects, the first 1D convolutional block can have a stride value of two.

The model may include an inception block. In some aspects, the inception block may include a number of sub-blocks. Each sub-block may include a number of convolutional blocks. For example, the each sub-block may include a first convolutional block, a second convolutional block, and a third convolutional block. The inception block may include four sub-blocks in series, such that the output of each sub-block is the input to the next sub-block. Each inception sub-block can generate and output a downsampled set of time-series information. Each sub-block can be configured with filters and filter windows as shown in the inception block with associated layer definition parameters.

In some aspects, the first convolutional block, the second convolutional block, and the third convolutional block can be 1D convolutional blocks. Results from each of the convolutional blocks can be concatenated by combining the results (e.g., arrays), and inputting the concatenated results to a MaxPool layer included in the sub-block. The MaxPool layer can extract positive values for each moving 1D convolutional filter window, and allows for another form of regularization, model generalization, and prevent overfitting. After completion of all four inception block processes, the output is passed to a final convolutional block and then a global average pooling (GAP) layer. The purpose of the GAP layer is to average the final downsampled ECG features from all eight independent ECG leads into a single downsampled array. The output of the GAP layer can be passed into the series of dense layer components (e.g., at the dense layer component). Furthermore, optimization parameters can also be set for all layers. For example, all layer parameters can enforce a kernel constraint parameter (max_norm=3), to prevent overfitting the model. The first convolutional block and the final convolutional block can utilize a stride parameter of n=1, whereas each inception block can utilize a stride parameter of n=2. The stride parameters determine the movement of every convolutional layer across the ECG time series and can have an impact on model performance.

In some aspects, the model can also concatenate supplementary data such as age and sex as described above, and the model can utilize the same dense layer component architecture as the model. The model can output a risk score based on the demographic information and the ECG information. Specifically, the dense layer components can output the risk score. In some aspects, the risk score can be indicative of a likelihood the patient will suffer from a condition within a predetermined period of time from when electrocardiogram data (e.g., the voltage data from the leads) was generated. In some aspects, the condition can be AF, mortality, ST-Elevation Myocardial Infarction (STEMI), Acute coronary syndrome (ACS), stroke, or other conditions indicated herein. In some aspects, the model can be trained to predict the risk of a patient developing AF in a predetermined time period following the acquisition of an ECG based on the ECG. In some aspects, the time period can range from one day to thirty years. For example, the time period may be one day, three months, six months, one year, five years, ten years, and/or thirty years.

### Additional endpoints and EHR data

In instances where additional data may inform the model, extraction may include records from electronic health records having additional patient data such as patient status (alive / dead) which may be generated by combining each patient's most recent clinical encounters from the EHR and a regularly-updated death index registry. Patient status is used as an endpoint to determine predictions for 1-year mortality after an ECG, however, additional clinical outcomes may also be predicted, including, but not limited to, mortality at any interval (1, 2, 3 years, etc.); mortality associated with heart disease, cardiovascular disease, sudden cardiac death; hospitalization for cardiovascular disease; need for intensive care unit admission for cardiovascular disease; emergency department visit for cardiovascular disease; new onset of an abnormal heart rhythm such as atrial fibrillation; need for a heart transplant; need for an implantable cardiac device such as a pacemaker or defibrillator; need for mechanical circulatory support such as a left ventricular / right ventricular / biventricular assist device or a total artificial heart; need for a significant cardiac procedure such as percutaneous coronary intervention or coronary artery bypass graft / surgery; new stroke or transient ischemic attack; new acute coronary syndrome; or new onset of any form of cardiovascular disease such as heart failure; or the likelihood of diagnosis from other diseases which may be informed from an ECG.

Moreover, additional variables may be added into a predictive model for purposes of both improving the prediction accuracy of the endpoints and identifying treatments which can positively impact the predicted bad outcome. For example, by extracting laboratory values (blood cholesterol measurements such as LDL / HDL / total cholesterol, blood counts such as hemoglobin / hematocrit / white blood cell count, blood chemistries such as glucose / sodium / potassium / liver and kidney function labs, and additional cardiovascular markers such as troponins and natriuretic peptides), vital signs (blood pressures, heart rate, respiratory rate, oxygen saturation), imaging metrics (such as cardiac ejection fractions, cardiac chamber volumes, heart muscle thickness, heart valve function), patient diagnoses (such as diabetes, chronic kidney disease, congenital heart defects, cancer, etc.) and treatments (including procedures, medications, referrals for services such as cardiac rehabilitation, dietary counseling, etc.), a model's accuracy may be improved. Some of these variables are "modifiable" risk factors that can then be used as inputs to the models to demonstrate the benefit of using a particular therapy. For example, a prediction may identify a patient as a 40% likelihood of developing atrial fibrillation in the next year, however, if the model was able to identify that the patient was taking a beta blocker, the predicted risk would drop to 20% based on the increased data available to the predictive model. In one example, demographic data and patient data may be supplemented with these additional variables, such as the extracted laboratory values or modifiable risk factors.

Machine learning models for implementing a predictive model may include a convolutional neural network having a plurality of branches processing a plurality of channels each. In some aspects, the model may include five branches from which an input of three leads as channels concurrent in time, e.g., (Branch 1: [I, II, III]; Branch 2: [aVR, aVL, aVF]; Branch 3: [V1, V2, V3]; Branch 4: [V4, V5, V6] and Branch 5: [V1-long, II-long, V5-long]) may be utilized to generate predictions. In some multi-branch CNNs, each branch can represent the 3 leads as they were acquired at the same time, or during the same heartbeats. For Branch 5, which may include the "long leads," the leads can be sampled for a duration of 10 seconds. For the other four branches, the leads can be sampled for a duration of 2.5 seconds.

In a typical 12-lead ECG, four of these branches of 3 leads are acquired over a duration of 10 seconds. Concurrently, the "long leads" are recorded over the entire 10 second duration. To improve robustness of the CNN, an architecture may be designed to account for these details since abnormal heart rhythms, in particular, cause the traces to change morphology throughout the standard 10 second clinical acquisition. A traditional model may miss abnormal heart rhythms which present with morphology deviations during a longer, 10-second read.

A convolutional block may include a 1-dimensional convolution layer followed by batch normalization and rectified linear units (ReLU) activations. In one example, the first four branches and last branch may include 4 and 6 convolutional blocks, respectively, followed by a Global Average Pooling (GAP) layer. The outputs of all the branches may then be concatenated and connected to a series of dense layers, such as a series of six layers, including layers having 256 (with dropout), 128 (with dropout), 64, 32, 8 and 1 unit(s) with a sigmoid function as the final layer. An Adam optimizer with a learning rate of 1e-5 and batch size of 2048 may be computed for each model branch in parallel on a separate GPU for faster computation. Additional architectures may include (1) replacing the GAP layer with recurrent neural networks such as long short-term memory and gated recurrent units; (2) changing the number of convolutional layers with varying filter sizes in all or number of branches in the present architecture or in addition, changing the number of branches in the architecture; (3) addition of derived signals from the time-voltage traces such as power spectral densities to the model training; and (4) addition of tabular or derived features from EHR such as laboratory values, echo measurements, ICD codes, and/or care gaps in addition to age and sex. In one example, demographic data and patient data may be supplemented with these additional tabular or derived features from the EHR of the subject.

### Training method

The training data may be divided into a plurality of folds with a last fold set aside as a validation set. An exemplary distribution may include five folds with five percent of the training data set aside as a validation set. The data may be split such that the same patient is not in both training and testing sets for cross-validation. The outcomes may be approximately balanced in the validation set. Training timing may be based upon validation loss which may be evaluated upon each training interval. Evaluated loss (binary cross-entropy) on the validation set for each epoch may be sufficient as a criterion. For example, training may be terminated if the validation loss fails to decrease for 10 epochs (as an early-stopping criteria), and the maximum number of epochs may be set to 500. An exemplary model may be implemented using Keras with a TensorFlow backend in python and default training parameters may be used. In other aspects, other models, programming languages, and parameters may be used. If all leads are sampled for a single common time period (e.g., twelve leads sampled from 0-10 seconds), then a single branch of the abovementioned model may be used. Demographic variables may be added to the model to boost robustness and improve predictions. As an example, demographic variables of age and sex may be added to the model by concatenating with the other branches a 64 hidden unit layer following. In one example training may be performed on an NVIDIA DGX1 platform with eight V100 GPUs and 32 GB of RAM per GPU. Training, however, may be performed via any computing devices, CPUs, GPUs, FPGAs, ASICs, and the like with variations in duration based upon the available computer power available at each training device. In on example, fitting a fold on 5 GPUs and each epoch took approximately 10 minutes.

For additional external validation, it may be advantageous to utilize data acquired at a certain hospital or other provider for training, and then test the model on all data acquired at the other hospitals / other providers. Segmenting training and validation sets by institutions allows formation of an additional independent validation of model accuracy.

### Model Operation

Once a model is sufficiently trained, the model may be used to predict one or more status associated with a patient based on the patient's ECG. As such, inputs to the trained model include, at a minimum, an ECG. The model's accuracy may be increased, and as such add additional utility (e.g. with the capability to recommend treatment changes) by having additional clinical variable inputs as described in detail above.

Outputs of the trained model may include the likelihood of a future adverse outcome (potential outcomes are listed in detail above) and potential interventions that may be performed to reduce the likelihood of the adverse outcome. An exemplary intervention that may be suggested includes notifying the attending physician that if a patient receives a beta blocker medication, their risk of hospitalization may decrease from 10% to 5%.

Generating predictions from these models may include satisfying an objective to determine the future risk of an adverse clinical outcome, in order to ultimately assist clinicians and patients with earlier treatment and potentially even prevention as a result of the earlier intervention. The duration between the ECG and the ultimate prediction (for example 1 year in the case of predicting 1-year mortality) may vary depending on the clinical outcome of interest and the intervention that may ultimately be suggested and/or performed. As references above, the models may be trained for any relevant time duration after the ECG acquisition, such as a period of time including 1, 2, 3, 4 or 5 years (or more), and for any relevant clinical prediction. Additionally, for each relevant clinical prediction, an intervention may be similarly suggested based upon either a model learned correlation, or publications of interventions. An example may include predicting that a patient has a 40% chance of a-fib in the next year; however, if the patient is prescribed (and takes) a beta blocker, that same patient may instead have a reduced, 20% chance of developing a-fib in the next year. Incorporating precision medicine at the earliest stages in treatment, such as when the patient incurs a first ECG, allows treating physicians to make recommendations that may improve the patient's overall quality of life and prevent unfavorable outcomes before the patient's health deteriorates to the point where they seek advanced medical treatment. Furthermore, by incorporating additional variables above and beyond the ECG into the training phase of development, the models will learn how certain treatments / interventions can positively impact patient outcomes that is reduce the chance of the adverse clinical outcome of interest. During the operation phase, the model can ingest the ECG and any relevant clinical variable inputs and then output predicted likelihood of the adverse clinical outcome either without or with certain treatments / interventions. Even if the patient's current treatments are unknown, the model can make suggestions such as: "If this patient happens to be diabetic, then their chance of 1-year mortality is reduced by 10% if their blood glucose is adequately controlled according to clinical guidelines."

### Additional Exemplary Model Operations

In one aspect, a sufficiently trained model may predict likelihood of a-fib and include a further suggestion, based upon the patient's height, weight, or BMI, that weight loss is needed to improve the patient's overall response to therapy. A sufficiently trained model may include a model that ingests a PDF of a clinically-acquired 12-lead resting ECG and outputs the precise risk of mortality at 1 year as a likelihood ranging from 0 to 1 where the model also received a patient height, weight, or BMI and the patient's clinical updates over the course of at least a year.

A composite model is disclosed herein. In some aspects, the composite model is an ECG-based machine-learning composite model. In some aspects, the composite model can predict a composite heart disease endpoint or cardiac event. In some aspects, a composite model yields a higher PPV to facilitate more practical recommendation of echocardiography to improve under-diagnosis of heart disease. In some aspects, the composite model comprises an ECG-based machine learning approach to predict multiple heart disease endpoints simultaneously.

A composite model may be used, for example, to identify high-risk patients. The composite model may use data more ubiquitously available than TTEs, such as 12-lead ECGs. ECGs are far more common, inexpensive, and performed for a much broader range of indications, including on asymptomatic patients (for example in the preoperative setting). The composite model may thus serve as a screening tool such that patients identified as high risk could be referred for diagnostic TTE.

In some aspects, the composite model may be used to identify patients at high risk for any one of numerous heart disease endpoints within a single ECG platform, including moderate or severe valvular disease (AS, aortic regurgitation [AR], mitral stenosis [MS], mitral regurgitation [MR], tricuspid regurgitation [TR]), reduced left ventricular ejection fraction (EF), and increased interventricular septal (IVS) thickness. The composite model may generate a composite prediction with higher yield / PPV that would facilitate a more practical clinical recommendation for follow-up diagnostic echocardiography.

Clinically, a composite model can enable targeted TTE screening to help detect unrecognized and underdiagnosed diseases. A composite model may have both high sensitivity and precision. The composite model can help guide the decision to obtain a TTE even for asymptomatic patients, shifting the balance to a scenario where TTE can be effective as a screening tool downstream of an ECG, and helping clinicians diagnose patients at the right time to prevent downstream adverse events, optimize the timing of interventions, and better implement evidence-based monitoring or management.

A machine-learning composite model using only ECG-based inputs can predict multiple important cardiac endpoints within a single platform with both good performance and high PPV, thereby representing a practical tool with which to better target TTE to detect undiagnosed disease. As shown in Example 1, below, an exemplary composite model is described and confirmatory results through retrospective real-world deployment scenarios are provided, to show the large impact that such a model can have on patients when deployed across a health system. These approaches to both clinical predictions and simulated deployment represent practical solutions for existing limitations in the implementation of machine learning in healthcare.

In some aspects, the machine learning composite model may be trained to predict composite echocardiography-confirmed disease within a certain period of time. For example, the composite model may be trained to predict composite disease within 1 year. In some aspects, the machine learning composite model may be trained to predict 2, 3, 4, 5, 6, 7, or more diseases. For example, an exemplary composite model may be trained to predict moderate or severe valvular disease. As another example, a composite model may be trained to predict aortic stenosis, aortic regurgitation, mitral stenosis, mitral regurgitation, tricuspid regurgitation, abnormally reduced ejection fraction, and abnormal interventricular septal thickness.

### Example 1 - Retrofitting an Existing Multi-Network Architecture Into a Single Multimodal Network Architecture

An existing machine-learning platform may include a composite model which can effectively predict clinically significant valvular disease, reduced left ventricular EF, and increased septal thickness with excellent performance (AUROC 91.4%) by using only ECG traces, age, and sex. The model may be trained using 2,141,366 ECGs linked to structured echocardiography and electronic health record data from 461,466 adults to predict composite echocardiography-confirmed disease within 1 year. Seven exemplary diseases may be included in the composite label: moderate or severe valvular disease (aortic stenosis or regurgitation, mitral stenosis (MS) or regurgitation, tricuspid regurgitation), reduced ejection fraction (EF) <50%, or interventricular septal thickness > 15mm.

### ECG labeling

An ECG may be labeled as positive for a given outcome if it was acquired up to one year before or any time after (up to a censoring event) the patient's first positive TTE report. An ECG was labeled as negative if it was acquired more than one year prior to the last negative TTE or a censoring event without any prior positive TTEs. Specifically, Figure 4d displays the patient timeline used to label (I) positive ECGs (+ECG in light gray background), (II) confirmed negative ECGs (-ECG in dark gray background), and (III) unconfirmed negative ECGs (-ECG in light gray background). The censoring event (A.I) and (A.II) are any intervention that could modify the underlying physiology of the disease of interest. The last negative Echo ensures no record of prior positive Echo exists. The bottom timeline is used for patients that never got an Echo. The censoring event in (A.III) is defined as the last known patient encounter where physical presence is required. A censoring event was defined as death, end of observation, or an intervention that directly treated the disease and could modify the underlying physiology or impact the ECG signal, such as valve replacement or repair. In other aspects, heart transplant or LVAD status, for example, may be included as censoring events. We also used a negative TTE report after a positive TTE report as a censoring event to account for the possibility of such interventions being performed outside of the healthcare system.

### The Retrograde Model

The resulting model included eight individual models using different combinations of multiple input sets including structured data (demographics, vitals, labs, structured ECG findings and measurements) and ECG voltage traces. The ECG trace models each include a low-parameter convolutional neural network (CNN) with 18,495 trainable parameters that consisted of six 1D CNN-Batch Normalization-ReLU (CBR) layer blocks followed by a two-layer multilayer perceptron and a final logistic output layer, as shown in the following table. The network contains a total of 18,945 trainable and 384 non-trainable parameters. Both Dropout layers were set at 25% drop rate. CBR is a brief notation for a sequence of 1D CNN, batch normalization, and ReLU layers.

| **Layer** | **Output Shape** | **#Parameter s** |
|---|---|---|
| Input | (5000,8) | 0 |
| Rescaling | (5000,8) | 0 |
| CBR-1 | (5000,16) | 656+64 |
| CBR-2 | (5000,16) | 1,296+64 |
| MaxPool1D | (1666,16) | 0 |
| CBR-3 | (1666,16) | 1,296+64 |
| CBR-4 | (1666,16) | 1,296+64 |
| MaxPool1D | (555,16) | 0 |
| CBR-5 | (555,16) | 1,296+64 |
| CBR-6 | (555,16) | 1,296+64 |
| MaxPool1D | (185,16) | 0 |
| CBR-7 | (185,16) | 1,296+64 |
| CBR-8 | (185,16) | 1,296+64 |
| MaxPool1D | (61,16) | 0 |
| CBR-9 | (61,16) | 1,296+64 |
| CBR-10 | (61,16) | 1,296+64 |
| MaxPool1D | (20,16) | 0 |
| CBR-11 | (20,16) | 1,296+64 |
| CBR-12 | (20,16) | 1,296+64 |
| MaxPool1D | (6,16) | 0 |
| Flatten | (96,) | 0 |
| Dense + Dropout | (32,) | 3104 |
| Dense + Dropout | (16,) | 528 |
| Dense | (1.) | 17 |

Each CNN layer consisted of 16 kernels of size 5. We used the same network configuration to train one model per clinical outcome, resulting in seven independently trained CNN models. A composite model may include a classification pipeline for ECG trace and other EHR data. The output of each neural network applied to ECG trace data is concatenated to labs, vitals, and demographics to form a feature vector. The vector is the input to the classification pipeline (min-max scaling, mean imputation, and XGBoost classifier), which outputs a recommendation score for the patient. To form the final composite model and combine ECG trace-based models with structured data, we concatenated the risk scores resulting from the individual CNNs with the structured data. We used the concatenated feature vector to train a classification pipeline consisting of a min-max scaler (min 0, max 1), mean imputation, and an XGBoost classifier, as shown in Figure 4D.

While examples provided herein include one or more combinations of model inputs, exemplary combinations of model inputs may be selected from any patient features within the EMR.

### Additional Considerations

Information and signals described herein may be represented using any of a variety of different technologies and techniques. For example, data, instructions, commands, information, signals, bits, symbols, and chips that may be referenced throughout the above description may be represented by voltages, currents, electromagnetic waves, magnetic fields or particles, optical fields or particles, or any combination thereof. Some drawings may illustrate signals as a single signal for clarity of presentation and description. It will be understood by a person of ordinary skill in the art that the signal may represent a bus of signals, wherein the bus may have a variety of bit widths and the disclosure may be implemented on any number of data signals including a single data signal.

The various illustrative logical blocks, modules, circuits, and algorithm acts described in connection with aspects disclosed herein may be implemented as electronic hardware, computer software, or combinations of both. To clearly illustrate this interchangeability of hardware and software, various illustrative components, blocks, modules, circuits, and acts are described generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of the aspects of the disclosure described herein.

In addition, it is noted that the aspects may be described in terms of a process that is depicted as a flowchart, a flow diagram, a structure diagram, or a block diagram. Although a flowchart may describe operational acts as a sequential process, many of these acts can be performed in another sequence, in parallel, or substantially concurrently. In addition, the order of the acts may be re-arranged. A process may correspond to a method, a function, a procedure, a subroutine, a subprogram, etc. Furthermore, the methods disclosed herein may be implemented in hardware, software, or both. If implemented in software, the functions may be stored or transmitted as one or more instructions or code on a computer-readable medium. Computer-readable media includes both computer storage media and communication media including any medium that facilitates transfer of a computer program from one place to another.

It should be understood that any reference to an element herein using a designation such as "first," "second," and so forth does not limit the quantity or order of those elements, unless such limitation is explicitly stated. Rather, these designations may be used herein as a convenient method of distinguishing between two or more elements or instances of an element. Thus, a reference to first and second elements does not mean that only two elements may be employed there or that the first element must precede the second element in some manner. Also, unless stated otherwise a set of elements may comprise one or more elements.

As used herein, the terms "component," "system" and the like are intended to refer to a computer-related entity, either hardware, a combination of hardware and software, software, or software in execution. For example, a component may be, but is not limited to being, a process running on a processor, a processor, an object, an executable, a thread of execution, a program, and/or a computer. By way of illustration, both an application running on a computer and the computer can be a component. One or more components may reside within a process and/or thread of execution and a component may be localized on one computer and/or distributed between two or more computers or processors.

The word "exemplary" is used herein to mean serving as an example, instance, or illustration. Any aspect or design described herein as "exemplary" is not necessarily to be construed as preferred or advantageous over other aspects or designs.

Furthermore, the disclosed subject matter may be implemented as a system, method, apparatus, or article of manufacture using standard programming and/or engineering techniques to produce software, firmware, hardware, or any combination thereof to control a computer or processor based device to implement aspects detailed herein. The term "article of manufacture" (or alternatively, "computer program product") as used herein is intended to encompass a computer program accessible from any computer-readable device, carrier, or media. For example, computer readable media may include but are not limited to magnetic storage devices (e.g., hard disk, floppy disk, magnetic strips ...), optical disks (e.g., compact disk (CD), digital versatile disk (DVD) . . .), smart cards, and flash memory devices (e.g., card, stick). Additionally, it should be appreciated that a carrier wave can be employed to carry computer-readable electronic data such as those used in transmitting and receiving electronic mail or in accessing a network such as the Internet or a local area network (LAN). Of course, those skilled in the art will recognize many modifications may be made to this configuration without departing from the scope or spirit of the claimed subject matter.

While the invention may be susceptible to various modifications and alternative forms, specific aspects have been shown by way of example in the drawings and have been described in detail herein. However, it should be understood that the invention is not intended to be limited to the particular forms disclosed.

Thus, the invention is to cover all modifications, equivalents, and alternatives falling within the spirit and scope of the invention as defined by the following appended claims.

To apprise the public of the scope of this invention, the following claims are made:

Further examples of the present invention are provided in the following.

Example 1: A computer-implemented method for configuring a single architecture deep learning model to process a plurality of dissimilar input feature modalities, the method comprising:
receiving predefined configuration parameters corresponding to three or more segments,
each respective segment corresponding to a different respective input feature modality,
each respective segment including one or more respective preprocessing layers, and
each respective segment including a respective plurality of isolated processing layers; configuring each segment based on the predefined configuration parameters;
for each respective segment:
   preprocessing the dissimilar input feature modalities using the respective preprocessing layers to generate respective preprocessing outputs, and
   processing the respective preprocessing outputs via the respective plurality of isolated processing layers to generate respective segment outputs, wherein the processing includes generating normalized output compatible with one or more other respective plurality of isolated processing layers; and
   processing the respective segment outputs via one or more combination layers to generate a combination output,
   wherein the combination output implements one or more loss functions, and
   wherein the combination output has an output type.

Example 2: The computer-implemented method of example 1, wherein the respective plurality of isolated processing layers implement at least one of:
I) an artificial neural network;
ii) a convolutional neural network; or
iii) a sequence neural network.

Example 3: The computer-implemented method of example 1, wherein receiving the predefined configuration parameters corresponding to the three or more segments includes receiving at least one of:
I) a digital image or a digital video input;
ii) a time series input;
iii) an electronic health records data input;
iv) an electrocardiogram input;
v) an echocardiogram input;
vi) a computed tomography input;
vii) a magnetic resonance imaging input;
viii) a tabular data input; or
ix) a natural language string input.

Example 4: The computer-implemented method of example 1,
wherein at least one of the plurality of dissimilar input feature modalities includes electrocardiogram time series data; and
wherein preprocessing the plurality of dissimilar input feature modalities via respective preprocessing layers to generate the respective preprocessing outputs includes preprocessing at least one of the dissimilar input feature modalities via:
I) an electrocardiogram lead recalibration;
ii) an electrocardiogram lead amplitude standardization;
iii) an electrocardiogram lead augmentation;
iv) an electrocardiogram data restructuring;
v) an electrocardiogram lead rescaling; or
vi) a Fourier space transformation.

Example 5: The computer-implemented method of example 1,
wherein at least one of the dissimilar input feature modalities includes tabular data; and
wherein preprocessing the dissimilar input feature modalities via the one or more respective preprocessing layers to generate the respective preprocessing outputs includes preprocessing at least one of the dissimilar input feature modalities via:
I) categorical feature processing;
ii) numerical feature processing; or
iii) feature censoring.

Example 6: The computer-implemented method of example 1,
wherein at least one of the dissimilar input feature modalities includes image and/or video data; and
wherein preprocessing the dissimilar input feature modalities via the one or more respective preprocessing layers to generate the respective preprocessing outputs includes preprocessing at least one of the dissimilar input feature modalities via:
I) image size normalization;
ii) image augmentation; or
iii) a Fourier space transformation.

Example 7: The computer-implemented method of example 1, further comprising:
receiving an expected format; and
wherein preprocessing the dissimilar input feature modalities via the one or more respective preprocessing layers to generate the respective preprocessing outputs is based on the expected format.

Example 8: The computer-implemented method of example 7, wherein the expected format includes one or more of:
(i) a single numeric value;
(ii) a range of numeric values; or
(iii) a Boolean value.

Example 9: The computer-implemented method of example 1, wherein the one or more loss functions include one or more of:
i) a regression function;
ii) a binary classification function;
iii) a multi-class classification function; or
iv) a survival modeling function.

Example 10: The computer-implemented method of example 9, wherein the regression function is a linear activation function included in one or more neurons of a dense output layer.

Example 11: The computer-implemented method of example 9, wherein the regression function includes one or more of:
i) a mean squared error loss function;
ii) a mean squared logarithmic error loss function;
iii) a mean absolute error loss function; or
iv) a Huber loss function.

Example 12: The computer-implemented method of example 9, wherein the binary classification function is a sigmoid activation function included in one or more neurons of a dense output layer.

Example 13: The computer-implemented method of example 9, wherein the binary classification function includes one or more of:
i) a binary cross entropy loss function;
ii) a Kullback-Leibler divergence loss function;
iii) a Hinge function loss function;
iv) a squared Hinge loss function; or
v) a focal loss function.

Example 14: The computer-implemented method of example 9, wherein the multi-class classification function is a softmax activation function included in one or more neurons of a dense output layer.

Example 15: The computer-implemented method of example 9, wherein the multi-class classification function includes one or more of:
i) a categorical cross entropy loss function;
ii) Kullback-Leibler divergence loss function;
iii) a sparse multiclass cross-entropy loss function;
iv) a focal loss function; or
v) a negative log-likelihood function.

Example 16: The computer-implemented method of example 9, wherein the survival modeling function is a linear activation function included in one or more neurons of a dense output layer.

Example 17: The computer-implemented method of example 9, wherein the survival modeling function includes a Cox-proportional hazard loss function.

Example 18: The computer-implemented method of example 1,
wherein the respective plurality of isolated processing layers include one or more binary classification layers and one or more final output layers;
wherein the loss functions include a binary classification loss function; and wherein the final output layers include a binary classification output layer.

Example 19: The computer-implemented method of example 1,
wherein the dissimilar input feature modalities include electrocardiogram features and electronic health record features; and
wherein the combination output is a probability from 0 and 1 representing a future risk to a patient.

Example 20: The computer-implemented method of example 1,
wherein the dissimilar input feature modalities include electrocardiogram features, echocardiogram features and electronic health record features; and
wherein the combination output is a probability from 0 and 1 representing a future risk to a patient.

Example 21: The computer-implemented method of example 1,
wherein the dissimilar input feature modalities include retinal scan image features and electronic healthcare record features; and
wherein the combination output is a probability from 0 and 1 representing a patient's future risk of onset diabetes.

Example 22: The computer-implemented method of example 1,
wherein the isolated processing layers include one or more binary classification layers and one or more final output layers;
wherein the loss functions include a regression loss function; and
wherein the combination layers include a regression output layer.

Example 23: The computer-implemented method of example 1,
wherein the dissimilar input feature modalities include electrocardiogram features and electronic healthcare record features; and
wherein the combination output is a numerical value indicating a physiological aspect of a patient.

Example 24: The computer-implemented method of example 1,
wherein the isolated processing layers include one or more survival modeling layers and one or more final output layers;
wherein the loss functions include a survival loss function; and
wherein the combination layers include a survival output layer.

Example 25: The computer-implemented method of example 1,
wherein the dissimilar input feature modalities include electrocardiogram features and electronic healthcare record features; and
wherein the combination output is a numerical value indicating a number of days until an event as a survival curve.

Example 26: The computer-implemented method of example 1,
wherein the dissimilar input feature modalities include genomic data features, echocardiogram features, electrocardiogram features and electronic healthcare record features; and
wherein the combination output is a numerical value indicating a number of days until an event as a survival curve.

Example 27: The computer-implemented method of example 1,
wherein the isolated processing layers include one or more multiclass classification layers and one or more final output layers;
wherein the loss functions include a multiclass classification loss function; and wherein the combination layers include a multiclass classification output layer.

Example 28: The computer-implemented method of example 1,
wherein the dissimilar input feature modalities include electrocardiogram features and electronic healthcare record features; and
wherein the combination output is a respective probability from 0 to 1 corresponding to a plurality of heart failure classifications.

Example 29: The computer-implemented method of example 1,
wherein the plurality of dissimilar input feature modalities includes a structured data set and a time series data set;
wherein preprocessing the dissimilar input feature modalities using the respective preprocessing layers to generate respective preprocessing outputs includes preprocessing the structured data set using at least one of (i) categorical feature processing, (ii) numerical feature processing, or (iii) feature censoring;
wherein preprocessing the dissimilar input feature modalities using the respective preprocessing layers to generate respective preprocessing outputs includes preprocessing the time series data set using at least one of (i) electrocardiogram lead recalibration, (ii) electrocardiogram lead amplitude standardization, (iii) electrocardiogram lead augmentation, (iv) electrocardiogram data restructuring, (v) electrocardiogram lead rescaling, or (vi) Fourier transformation; and wherein the one or more loss functions include a binary classification loss function that generates one of (i) a positive prediction, or (ii) a negative prediction.

Example 30: The computer-implemented method of example 29, wherein the structured data set is a tabular data set.

Example 31: A computing system for configuring a single architecture deep learning model to process a plurality of dissimilar input feature modalities, comprising: one or more processors; and
one or more memories having stored thereon computer-executable instructions that, when executed, cause the computing system to:
receive predefined configuration parameters corresponding to three or more segments, each respective segment corresponding to a different respective input feature modality, each respective segment including one or more respective preprocessing layers, and each respective segment including a respective plurality of isolated processing layers; configure each segment based on the predefined configuration parameters;
for each respective segment:
   preprocess the dissimilar input feature modalities using the respective preprocessing layers to generate respective preprocessing outputs, and
   process the respective preprocessing outputs via the respective plurality of isolated processing layers to generate respective segment outputs, wherein the processing includes generating normalized output compatible with one or more other respective plurality of isolated processing layers; and
   process the respective segment outputs via one or more combination layers to generate a combination output,
   wherein the combination output implements one or more loss functions, and
   wherein the combination output has an output type.

Example 32: A non-transitory computer-readable medium having stored thereon computer-executable instructions that, when executed by one or more processors, cause a computer to:
receive predefined configuration parameters corresponding to three or more segments, each respective segment corresponding to a different respective input feature modality, each respective segment including one or more respective preprocessing layers, and each respective segment including a respective plurality of isolated processing layers; configure each segment based on the predefined configuration parameters;
for each respective segment:
   preprocess the dissimilar input feature modalities using the respective preprocessing layers to generate respective preprocessing outputs, and
   process the respective preprocessing outputs via the respective plurality of isolated processing layers to generate respective segment outputs, wherein the processing includes generating normalized output compatible with one or more other respective plurality of isolated processing layers; and
   process the respective segment outputs via one or more combination layers to generate a combination output,
   wherein the combination output implements one or more loss functions, and
   wherein the combination output has an output type.

## Claims

1. A computer-implemented method for configuring a single architecture deep learning model to process a plurality of dissimilar input feature modalities, the method comprising:
receiving predefined configuration parameters corresponding to three or more segments,
each respective segment corresponding to a different respective input feature modality,
each respective segment including one or more respective preprocessing layers, and
each respective segment including a respective plurality of isolated processing layers;
configuring each segment based on the predefined configuration parameters;
for each respective segment:
preprocessing the dissimilar input feature modalities using the respective preprocessing layers to generate respective preprocessing outputs, and
processing the respective preprocessing outputs via the respective plurality of isolated processing layers to generate respective segment outputs, wherein the processing includes generating normalized output compatible with one or more other respective plurality of isolated processing layers; and
processing the respective segment outputs via one or more combination layers to generate a combination output,
wherein the combination output implements one or more loss functions, and
wherein the combination output has an output type.

2. The computer-implemented method of claim 1, wherein the respective plurality of isolated processing layers implement at least one of:
i) an artificial neural network;
ii) a convolutional neural network; or
iii) a sequence neural network.

3. The computer-implemented method of claim 1 or 2, wherein receiving the predefined configuration parameters corresponding to the three or more segments includes receiving at least one of:
i) a digital image or a digital video input;
ii) a time series input;
iii) an electronic health records data input;
iv) an electrocardiogram input;
v) an echocardiogram input;
vi) a computed tomography input;
vii) a magnetic resonance imaging input;
viii) a tabular data input; or
ix) a natural language string input.

4. The computer-implemented method of one of the previous claims,
wherein at least one of the plurality of dissimilar input feature modalities includes electrocardiogram time series data; and
wherein preprocessing the plurality of dissimilar input feature modalities via respective preprocessing layers to generate the respective preprocessing outputs includes preprocessing at least one of the dissimilar input feature modalities via:
i) an electrocardiogram lead recalibration;
ii) an electrocardiogram lead amplitude standardization;
iii) an electrocardiogram lead augmentation;
iv) an electrocardiogram data restructuring;
v) an electrocardiogram lead rescaling; or
vi) a Fourier space transformation.

5. The computer-implemented method of one of the previous claims,
wherein at least one of the dissimilar input feature modalities includes tabular data; and
wherein preprocessing the dissimilar input feature modalities via the one or more respective preprocessing layers to generate the respective preprocessing outputs includes preprocessing at least one of the dissimilar input feature modalities via:
i) categorical feature processing;
ii) numerical feature processing; or
iii) feature censoring, and/or wherein at least one of the dissimilar input feature modalities includes image and/or video data; and
wherein preprocessing the dissimilar input feature modalities via the one or more respective preprocessing layers to generate the respective preprocessing outputs includes preprocessing at least one of the dissimilar input feature modalities via:
i) image size normalization;
ii) image augmentation; or
iii) a Fourier space transformation.

6. The computer-implemented method of one of the previous claims, further comprising:
receiving an expected format; and
wherein preprocessing the dissimilar input feature modalities via the one or more respective preprocessing layers to generate the respective preprocessing outputs is based on the expected format, preferably wherein the expected format includes one or more of:
(i) a single numeric value;
(ii) a range of numeric values; or
(iii) a Boolean value.

7. The computer-implemented method of one of the previous claims, wherein the one or more loss functions include one or more of:
i) a regression function;
ii) a binary classification function;
iii) a multi-class classification function; or
iv) a survival modeling function, wherein preferably the regression function is a linear activation function included in one or more neurons of a dense output layer, and/or wherein the regression function includes one or more of:
i) a mean squared error loss function;
ii) a mean squared logarithmic error loss function;
iii) a mean absolute error loss function; or
iv) a Huber loss function, and/or wherein the binary classification function is a sigmoid activation function included in one or more neurons of a dense output layer, and/or wherein the binary classification function includes one or more of:
i) a binary cross entropy loss function;
ii) a Kullback-Leibler divergence loss function;
iii) a Hinge function loss function;
iv) a squared Hinge loss function; or
v) a focal loss function.

8. The computer-implemented method of claim 7, wherein the multi-class classification function is a softmax activation function included in one or more neurons of a dense output layer, and/or wherein the multi-class classification function includes one or more of:
i) a categorical cross entropy loss function;
ii) Kullback-Leibler divergence loss function;
iii) a sparse multiclass cross-entropy loss function;
iv) a focal loss function; or
v) a negative log-likelihood function.

9. The computer-implemented method of claim 7, wherein the survival modeling function is a linear activation function included in one or more neurons of a dense output layer, and/or wherein the survival modeling function includes a Cox-proportional hazard loss function.

10. The computer-implemented method of one of the previous claims,
wherein the dissimilar input feature modalities include electrocardiogram features and electronic health record features; and
wherein the combination output is a probability from 0 and 1 representing a future risk to a patient, and/or wherein the dissimilar input feature modalities include electrocardiogram features, echocardiogram features and electronic health record features; and
wherein the combination output is a probability from 0 and 1 representing a future risk to a patient, and/or wherein the dissimilar input feature modalities include retinal scan image features and electronic healthcare record features; and
wherein the combination output is a probability from 0 and 1 representing a patient's future risk of onset diabetes, and/or
wherein the dissimilar input feature modalities include electrocardiogram features and electronic healthcare record features; and
wherein the combination output is a numerical value indicating a physiological aspect of a patient, and/or
wherein the dissimilar input feature modalities include electrocardiogram features and electronic healthcare record features; and
wherein the combination output is a numerical value indicating a number of days until an event as a survival curve, and/or wherein the dissimilar input feature modalities include genomic data features, echocardiogram features, electrocardiogram features and electronic healthcare record features; and
wherein the combination output is a numerical value indicating a number of days until an event as a survival curve, and/or
wherein the dissimilar input feature modalities include electrocardiogram features and electronic healthcare record features; and
wherein the combination output is a respective probability from 0 to 1 corresponding to a plurality of heart failure classifications.

11. The computer-implemented method of one of the previous claims,
wherein the isolated processing layers include one or more multiclass classification layers and one or more final output layers;
wherein the loss functions include a multiclass classification loss function; and
wherein the combination layers include a multiclass classification output layer, and/or
wherein the isolated processing layers include one or more survival modeling layers and one or more final output layers;
wherein the loss functions include a survival loss function; and
wherein the combination layers include a survival output layer,
and/or
wherein the respective plurality of isolated processing layers include one or more binary classification layers and one or more final output layers;
wherein the loss functions include a binary classification loss function; and
wherein the final output layers include a binary classification output layer, and/or
wherein the isolated processing layers include one or more binary classification layers and one or more final output layers;
wherein the loss functions include a regression loss function; and
wherein the combination layers include a regression output layer.

12. The computer-implemented method of one of the previous claims,
wherein the plurality of dissimilar input feature modalities includes a structured data set and a time series data set;
wherein preprocessing the dissimilar input feature modalities using the respective preprocessing layers to generate respective preprocessing outputs includes preprocessing the structured data set using at least one of (i) categorical feature processing, (ii) numerical feature processing, or (iii) feature censoring;
wherein preprocessing the dissimilar input feature modalities using the respective preprocessing layers to generate respective preprocessing outputs includes preprocessing the time series data set using at least one of (i) electrocardiogram lead recalibration, (ii) electrocardiogram lead amplitude standardization, (iii) electrocardiogram lead augmentation, (iv) electrocardiogram data restructuring, (v) electrocardiogram lead rescaling, or (vi) Fourier transformation; and
wherein the one or more loss functions include a binary classification loss function that generates one of (i) a positive prediction, or (ii) a negative prediction.

13. The computer-implemented method of claim 12, wherein the structured data set is a tabular data set.

14. A computing system for configuring a single architecture deep learning model to process a plurality of dissimilar input feature modalities, comprising:
one or more processors; and
one or more memories having stored thereon computer-executable instructions that, when executed, cause the computing system to:
receive predefined configuration parameters corresponding to three or more segments,
each respective segment corresponding to a different respective input feature modality,
each respective segment including one or more respective preprocessing layers, and
each respective segment including a respective plurality of isolated processing layers;
configure each segment based on the predefined configuration parameters;
for each respective segment:
preprocess the dissimilar input feature modalities using the respective preprocessing layers to generate respective preprocessing outputs, and
process the respective preprocessing outputs via the respective plurality of isolated processing layers to generate respective segment outputs, wherein the processing includes generating normalized output compatible with one or more other respective plurality of isolated processing layers; and
process the respective segment outputs via one or more combination layers to generate a combination output,
wherein the combination output implements one or more loss functions, and
wherein the combination output has an output type.

15. A non-transitory computer-readable medium having stored thereon computer-executable instructions that, when executed by one or more processors, cause a computer to:
receive predefined configuration parameters corresponding to three or more segments,
each respective segment corresponding to a different respective input feature modality,
each respective segment including one or more respective preprocessing layers, and
each respective segment including a respective plurality of isolated processing layers;
configure each segment based on the predefined configuration parameters;
for each respective segment:
preprocess the dissimilar input feature modalities using the respective preprocessing layers to generate respective preprocessing outputs, and
process the respective preprocessing outputs via the respective plurality of isolated processing layers to generate respective segment outputs, wherein the processing includes generating normalized output compatible with one or more other respective plurality of isolated processing layers; and
process the respective segment outputs via one or more combination layers to generate a combination output,
wherein the combination output implements one or more loss functions, and
wherein the combination output has an output type.
